# EUROPEAN PATENT SPECIFICATION

(11) **EP 4 555 933 B1**
(45) Date of publication and mention of the grant of the patent: **16.09.2026**
(21) Application number: 23856132.8
(22) Date of filing: 12.05.2023
(51) Int. Cl.: A61B 5/369

(54) **METHOD FOR DETECTING TIME PHASE OF EEG SIGNAL, TIME PHASE DETECTION UNIT, AND CLOSED-LOOP REGULATION AND CONTROL SYSTEM**
VERFAHREN ZUR ERKENNUNG DER ZEITPHASE EINES EEG-SIGNALS, ZEITPHASENERKENNUNGSEINHEIT UND REGEL- UND STEUERUNGSSYSTEM MIT GESCHLOSSENEM REGELKREIS
PROCÉDÉ DE DÉTECTION DE PHASE TEMPORELLE DE SIGNAL EEG, UNITÉ DE DÉTECTION DE PHASE TEMPORELLE, ET SYSTÈME DE RÉGULATION ET DE COMMANDE EN BOUCLE FERMÉE

(30) Priority: 22.08.2022 CN 202211006518
(43) Date of publication of application: 21.05.2025
(73) Proprietor: Neuracle Technology (Shanghai) Co., Ltd., Shanghai (CN)
(72) Inventor: HUANG, Xiaoshan, hangzhou, Jiangsu 213164 (CN); XU, Honglai, hangzhou, Jiangsu 213164 (CN); ZHANG, Xirui, hangzhou, Jiangsu 213164 (CN); LI, Hanlei, hangzhou, Jiangsu 213164 (CN); LU, Lei, hangzhou, Jiangsu 213164 (CN); FANG, Xiang, hangzhou, Jiangsu 213164 (CN); LIU, Weixiang, hangzhou, Jiangsu 213164 (CN); LIANG, Xing, hangzhou, Jiangsu 213164 (CN)
(74) Representative: Lorenz Seidler Gossel Part. mbB
(86) International application number: PCT/CN2023/093731
(87) International publication number: WO 2024/041038

(56) References cited:
- CN-A- 111 329 469
- CN-A- 112 043 473
- CN-A- 112 842 358
- CN-A- 113 499 079
- CN-A- 114 098 667
- CN-A- 114 145 723
- CN-A- 114 469 138
- CN-A- 114 470 516
- CN-A- 114 582 485
- CN-A- 115 089 196

## Description

### TECHNICAL FIELD

The present disclosure relates to the technical field of signal detection, and in particular, to a time-phase detection method for an online signal, a time-phase detection unit, and a closed-loop regulation and control system.

### BACKGROUND

A time-domain signal is a segment of data whose signal feature changes over time. An electroencephalogram (EEG) signal is also a segment of time-domain signal. Currently, the EEG signal is mostly analyzed through frequency-domain analysis, time-domain analysis, wavelet analysis, and other methods. These methods have a low detection rate for an abnormal signal and often result in missed detection. For example, CN 114469138 A shows a method for detecting burst suppression patterns in EEG signals, wherein using several feature parameters is suggested including envelope, envelope derivate and energy in different frequency bands. Furthermore, document CN 114470516 A discloses a control method for a stimulation device, wherein a library of historical signal propagation patterns is matched with a real-time event to select an appropriate stimulation response.

### SUMMARY

The present disclosure is intended to solve a technical problem of low accuracy of a detection result of a detection method in the prior art. The present disclosure provides a time-phase detection method for an online signal, a time-phase detection unit, and a closed-loop regulation and control system, which adopt a plurality of detectors to detect an online signal, thereby improving accuracy of signal detection and preventing missed detection.

The technical solutions used in the present disclosure to solve the technical problem are as follows: A time-phase detection method for an online signal which is an EEG signal, includes: using one or more detectors to run a detection algorithm and detect an online signal in terms of an EEG signal in parallel, to output time-phase detection results of the online signal; and
determining a time phase of the online signal based on each of the time-phase detection results, where
the time-phase detection results of the online signal include an abnormal period and a normal period;
the abnormal period includes at least one of a continuous abnormal period with a sudden amplitude change, a continuous abnormal period with a gradual amplitude change, and an instantaneous abnormal period; and
the detection algorithm includes at least one of a classification algorithm based on a minimum window and an early-warning algorithm based on a zero-crossing coefficient.

Further, a number of the one or more detectors is one, namely a first detector is provided; and
the first detector adopts the classification algorithm based on the minimum window to output the normal period, the continuous abnormal period with the sudden amplitude change, the instantaneous abnormal period, or the continuous abnormal period with the gradual amplitude change in the time-phase detection result.

Further, a number of the one or more detectors is at least two; and
when the time-phase detection results of each of the detectors are all abnormal periods, the time phase of the online signal is determined based on severity grades or occurrence frequencies of the abnormal periods, where the severity grades of the abnormal periods are as follows: a severity grade of the continuous abnormal period with the sudden amplitude change > a severity grade of the continuous abnormal period with the gradual amplitude change > a severity grade of the instantaneous abnormal period;
when the time-phase detection results of each of the detectors include both the normal period and the abnormal period, a specified first logical operation is selected to determine the time phase of the online signal, where the first logical operation includes any one of an "OR" operation, an "AND" operation, a "NOR" operation, and a "NAND" operation; and the first logical operation is specified based on a collection region where the online signal is located.

Further, a number of the one or more detectors is two, namely a first detector and a second detector are provided, where
the first detector adopts the classification algorithm based on the minimum window to output the normal period, the continuous abnormal period with the sudden amplitude change, or the instantaneous abnormal period in the time-phase detection result; and
the second detector adopts the classification algorithm based on the minimum window or the early-warning algorithm based on the zero-crossing coefficient to output the normal period or the continuous abnormal period with the gradual amplitude change in the time-phase detection result.

Further, a number of the one or more detectors is three, namely a first detector, a second detector, and a third detector are provided, where
the first detector adopts the classification algorithm based on the minimum window to output the normal period or the continuous abnormal period with the sudden amplitude change in the time-phase detection result;
the second detector adopts the classification algorithm based on the minimum window to output the normal period or the instantaneous abnormal period in the time-phase detection result; and
the third detector adopts the classification algorithm based on the minimum window or the early-warning algorithm based on the zero-crossing coefficient to output the normal period or the continuous abnormal period with the gradual amplitude change in the time-phase detection result.

Further, the classification algorithm based on the minimum window includes:
dividing the online signal into a plurality of minimum window segments, and calculating signal eigenvalues of the plurality of minimum window segments to obtain a detection result of a single minimum window segment;
selecting detection results of N minimum window segments to obtain a determining result of a detection window segment; and
detecting the time phase of the online signal based on the determining result of at least one detection window segment, where
a window width of the minimum window segments is less than or equal to a duration of an instantaneous abnormal period in an offline signal, and a window width of the detection window segment is greater than the duration of the instantaneous abnormal period in the offline signal.

Further, the detection result of the single minimum window segment is obtained by:
marking a time phase of the offline signal;
obtaining a threshold indicator corresponding to at least one time phase; and
classifying the single minimum window segment, that is, comparing the single minimum window segment in the online signal with a threshold indicator corresponding to any one time phase, to classify the single minimum window segment as a normal minimum window or an abnormal minimum window, where
the threshold indicator includes at least one of a signal feature threshold and a feature difference threshold;
the classifying the single minimum window segment includes:
   comparing a signal eigenvalue of the single minimum window segment in the online signal with the signal feature threshold, and classifying the single minimum window segment as the abnormal minimum window when the signal eigenvalue of the single minimum window segment is greater than or equal to the signal feature threshold; or
   comparing a signal feature difference of the single minimum window segment with the feature difference threshold, and classifying the single minimum window segment as the abnormal minimum window when the signal feature difference of the single minimum window segment is greater than or equal to the feature difference threshold; or
   comparing the signal eigenvalue of the single minimum window segment with the feature threshold, and comparing the signal feature difference of the single minimum window segment with the feature difference threshold; and classifying the single minimum window segment as the abnormal minimum window when the signal eigenvalue of the single minimum window segment is greater than or equal to the signal feature threshold and/or the signal feature difference of the single minimum window segment is greater than or equal to the feature difference threshold; and
   the obtaining the threshold indicator corresponding to the at least one time phase includes:
      based on an offline signal marked as the normal period, obtaining a change curve P1 of a quantity proportion of detected minimum window segments of the offline signal under different feature thresholds or feature difference thresholds;
      based on an offline signal marked as the "instantaneous abnormal period", obtaining a change curve P2 of a quantity proportion of detected minimum window segments of the offline signal under the different feature thresholds or the feature difference thresholds;
      based on an offline signal marked as the "continuous abnormal period with the sudden amplitude change", obtaining a change curve P3 of a quantity proportion of detected minimum window segments of the offline signal under the different feature thresholds or the feature difference thresholds;
      based on an offline signal marked as the "continuous abnormal period with the gradual amplitude change", obtaining a change curve P4 of a quantity proportion of detected minimum window segments of the offline signal under the different feature thresholds or the feature difference thresholds; and
      obtaining corresponding feature thresholds of different time phases based on the change curve P1, a difference curve of the change curves P2 and P1, a difference curve of the change curves P3 and P1, and a difference curve of the change curves P4 and P1, where
      a signal feature threshold corresponding to the instantaneous abnormal period is a specified value of the change curve P1, a maximum difference of a P2-P1 curve, or a maximum difference of a P2÷P1 curve;
      a signal feature threshold corresponding to the continuous abnormal period with the sudden amplitude change is the specified value of the change curve P1, a maximum difference of a P3-P1 curve, or a maximum difference of a P3÷P1 curve; and
      a signal feature threshold corresponding to the continuous abnormal period with the gradual amplitude change is the specified value of the change curve P1, a maximum difference of a P4-P1 curve, or a maximum difference of a P4÷P1 curve.

Further, the selecting the detection results of the N minimum window segments to obtain the determining result of the detection window segment includes:
taking statistics on a quantity of abnormal minimum window segments detected within the detection window segment; and
comparing numerical relationships between the quantity of the abnormal minimum window segments detected and each of M₁, M₂, and N by using a classification method, to obtain the determining result of the detection window segment, where
the M₁ is a threshold for a quantity of minimum windows obtained through traversal and selection in the continuous abnormal period with the sudden amplitude change, and the M₂ is a threshold for a quantity of minimum windows obtained through the traversal and selection in the instantaneous abnormal period; where
a method for the traversal and selection includes:
   performing traversal within a range of (0, N) to obtain a value of the threshold for the quantity of the minimum windows;
   separately calculating a detection performance indicator of the threshold for the quantity of the minimum windows under each value;
   respectively selecting, based on the detection performance indicator, the threshold M₁ for the quantity of the minimum windows in the continuous abnormal period with the sudden amplitude change and the threshold M₂ for the quantity of the minimum windows in the instantaneous abnormal period; and
   the detection performance indicator includes at least one of a false detection rate and a detection delay, where
   the false detection rate < 1 time/hour; and
   the detection delay < 12 seconds;
   the classification method includes at least one of a synchronous three-way classification method and an asynchronous three-way classification method;
   the synchronous three-way classification method is to perform threshold comparison once to obtain the quantity M of the abnormal minimum window segments detected within the detection window segment, and compare numerical relationships between the M and each of the M₁, the M₂, and the N at the same time, where
   when M₁≤M≤N, the determining result of the detection window segment indicates the continuous abnormal period with the sudden amplitude change;
   when M₂≤M<M₁, the determining result of the detection window segment indicates the instantaneous abnormal period;
   when 0≤M<M₂, the determining result of the detection window segment indicates the normal period;
   the asynchronous three-way classification is to perform the threshold comparison twice to respectively obtain quantities K₁ and K₂ of the abnormal minimum window segments detected within the detection window segment, and compare numerical relationships between the K₁ and each of the M₁ and the N, as well as numerical relationships between the K₂ and each of M₂ and the N in parallel; and when different determining results are obtained for a same detection window segment, a second logical operation is used for secondary differentiation, where priorities of the second logical operation in the continuous abnormal period with the sudden amplitude change, the instantaneous abnormal period, and the normal period are in a descending order.

Further, the detecting the time phase of the online signal based on the determining result of the at least one detection window segment includes:
after obtaining determining results of a plurality of detection window segments, performing determining based on a k-of-n judgment principle, where
in a scenario of preferentially pursuing detection specificity, the k-of-n judgment principle requires that k detection window segments are detected continuously; and
in a scenario of preferentially pursuing detection sensitivity, the k-of-n judgment principle requires that the k detection window segments are detected discontinuously, where
n represents a quantity of detection window segments contained in the online signal, and k represents a quantity of detection window segments detected among n detection window segments.

Further, the early-warning algorithm based on the zero-crossing coefficient includes:
dividing the online signal into a plurality of windows to obtain a plurality of data segments;
extracting a signal eigenvalue for each of the plurality of data segments separately, and inputting the signal eigenvalue into a classifier to obtain a classification result of each of the plurality of data segments; and
if classification results of X consecutive data segments each are the continuous abnormal period with the gradual amplitude change, determining that the online signal is in the continuous abnormal period with the gradual amplitude change.

Further, when at least two detectors run two different detection algorithms and the two different detection algorithms have signal eigenvalues obtained through a same linear operation, the extracted signal eigenvalue of each of the plurality of data segments in the early-warning algorithm based on the zero-crossing coefficient is set as a sum of signal eigenvalues of the minimum window segments of the detection window segment in the classification algorithm based on the minimum window, where the signal eigenvalues each include at least one of the zero-crossing coefficient, a line length, and a curvature.

The present disclosure further provides a time-phase detection unit. The time-phase detection unit adopts the above time-phase detection method for the online signal, and includes:
a detection component including a plurality of detectors configured to output detection results corresponding to different time phases; and
a processor configured to determine a time phase of the online signal based on the detection results corresponding to the different time phases.

The present disclosure also provides a closed-loop regulation and control system, including the above time-phase detection unit, and
a regulation and control module configured to perform closed-loop regulation and control based on a time phase of an online signal.

The present disclosure achieves the following advantages: The present disclosure adopts a plurality of detectors to simultaneously detect an online signal in terms of an EEG signal, and each detector can detect a waveform characteristic in a different time phase, achieving stronger specificity. This significantly improves accuracy of detecting the online signal, and achieves more precise closed-loop regulation and control.

### BRIEF DESCRIPTION OF THE DRAWINGS

The present disclosure will be further described below in conjunction with the accompanying drawings and embodiments.
FIG. 1 shows a working process of a time-phase detection method for an online signal according to the present disclosure;
FIG. 2a shows a waveform of an online signal according to the present disclosure;
FIG. 2b is an enlarged view of an instantaneous abnormal period in FIG. 2a;
FIG. 2c is an enlarged view of a continuous abnormal period with a gradual amplitude change in FIG. 2a;
FIG. 2d is an enlarged view of a continuous abnormal period with a sudden amplitude change in FIG. 2a;
FIG. 3 is a curve chart showing that a feature threshold changes with a proportion of a detected minimum window according to the present disclosure;
FIG. 4 is a curve chart showing that a feature difference threshold changes with a proportion of a detected minimum window according to the present disclosure;
FIG. 5 is a graph showing a change between a false detection rate and a threshold M₁ for a quantity of minimum windows according to the present disclosure;
FIG. 6 is a graph showing a change between a detection delay and a threshold M₁ for a quantity of minimum windows according to the present disclosure;
FIG. 7 shows a working process of synchronous three-way classification according to the present disclosure;
FIG. 8 shows a working process of asynchronous three-way classification according to the present disclosure;
FIG. 9a is a schematic diagram of a detection result according to Embodiment 1 of the present disclosure;
FIG. 9b is a schematic diagram of a detection result according to Embodiment 2 of the present disclosure;
FIG. 9c is a schematic diagram of a detection result according to Embodiment 3 of the present disclosure;
FIG. 10a is a schematic diagram of a detection result according to Embodiment 4 of the present disclosure;
FIG. 10b is a schematic diagram of a detection result according to Embodiment 5 of the present disclosure;
FIG. 10c is a schematic diagram of a detection result according to Embodiment 6 of the present disclosure;
FIG. 11 shows a working process of an early-warning algorithm based on a zero-crossing coefficient according to the present disclosure;
FIG. 12 shows a working process of a first detector and a second detector according to the present disclosure;
FIG. 13 shows a working process of a second detector according to the present disclosure; and
FIG. 14 is a schematic diagram of parallel detection by two detectors according to the present disclosure.

### DETAILED DESCRIPTION OF THE EMBODIMENTS

The present disclosure will be described in further detail below in combination with accompanying drawings. These accompanying drawings are all simplified schematic diagrams, which merely illustrate the basic structure of the present disclosure in a schematic manner, and thus only show the parts associated with the present disclosure.

In the description of the present disclosure, it should be understood that orientations or position relationships indicated by terms "central", "longitudinal", "transverse", "long", "wide", "thick", "upper", "lower", "front", "rear", "left", "right", "vertical", "horizontal", "top", "bottom", "inner", "outer", "clockwise", "anticlockwise", "axial", "radial", and "circumferential" are based on the orientations or position relationships shown in the accompanying drawings. These terms are merely intended to facilitate a simple description of the present disclosure, rather than to indicate or imply that the mentioned apparatus or elements must have a specific orientation or be constructed and operated in a specific orientation. Therefore, these terms may not be construed as a limitation to the present disclosure. In addition, features defined with "first" and "second" may explicitly or implicitly include one or more of the features. In the description of the present disclosure, unless otherwise specified, "a plurality of" means two or more.

In the description of the present disclosure, it should be noted that, unless otherwise clearly specified and limited, the terms such as "installation", "interconnection", and "connection" are intended to be understood in a broad sense. For example, the "connection" may be a fixed connection, a removable connection, or an integral connection; may be a mechanical connection or an electrical connection; may be a direct connection or an indirect connection using a medium; and may be a communication between two elements. Those of ordinary skill in the art may understand specific meanings of the foregoing terms in the present disclosure based on a specific situation.

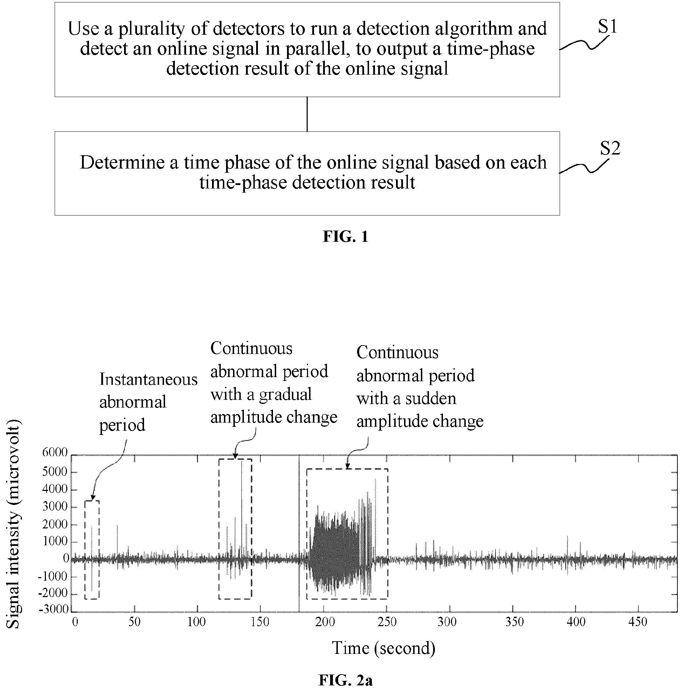

As shown in FIG. 1, a time-phase detection method for an online signal in the present disclosure includes the following steps: S1: One or more detectors are used to run a detection algorithm and detect an online signal in parallel, to output a time-phase detection result of the online signal. S2: A time phase of the online signal is determined based on each time-phase detection result. The time-phase detection result of the online signal includes an abnormal period and a normal period. The abnormal period includes at least one of a continuous abnormal period with a sudden amplitude change, a continuous abnormal period with a gradual amplitude change, and an instantaneous abnormal period. The detection algorithm includes at least one of a classification algorithm based on a minimum window and an early-warning algorithm based on a zero-crossing coefficient.

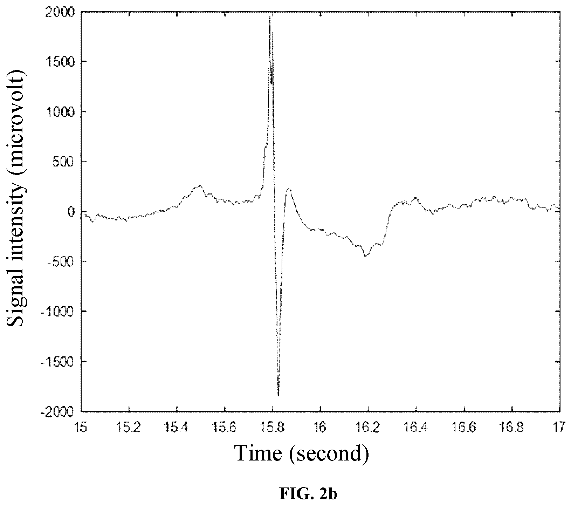

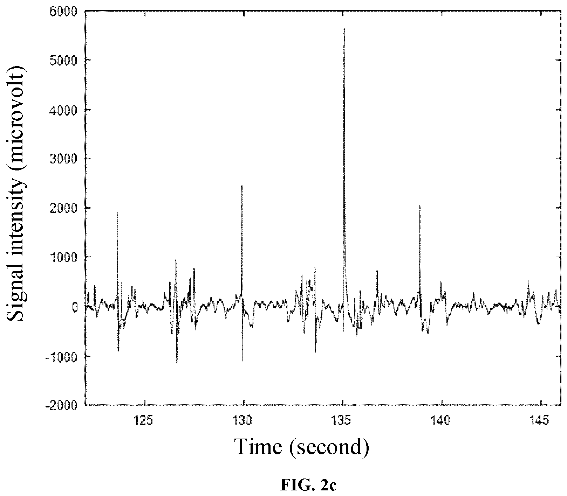

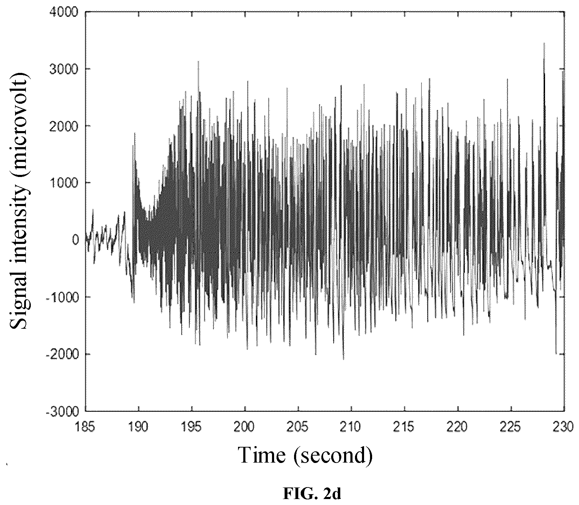

It should be noted that one segment of the online signal may contain a plurality of time phases (as shown in FIG. 2a to FIG. 2d), and waveform characteristics presented in different time phases may differ. If a same detection algorithm is used to detect the online signal, the detection result may be inaccurate, and some time phases cannot be detected. The present disclosure adopts a plurality of detectors to simultaneously detect an online signal, and each detector can detect a waveform characteristic in a different time phase, achieving stronger specificity. This significantly improves accuracy of detecting the online signal, and achieves more precise closed-loop regulation and control.

Specifically, in the embodiments of the present disclosure, one, at least two, or at least three detectors may be adopted, depending on actual needs. Core logic for selecting a quantity of detectors in the present disclosure is that the quantity of detectors corresponds to a quantity of types of the abnormal period in the time phase. Based on this, it is preferable to adopt one detector for time phases of a plurality of abnormal periods to reduce a computational amount and improve computational efficiency. When the present disclosure selects two detectors, one detector uses the classification algorithm based on the minimum window to pursue detection time resolution for the instantaneous abnormal period and the continuous abnormal period with the sudden amplitude change, and the other detector uses the early-warning algorithm based on the zero-crossing coefficient to pursue detection specificity for the continuous abnormal period with the gradual amplitude change. In this way, both detection accuracy of a plurality of time phases and high computational efficiency can be achieved. When three detectors are selected, logic in which one detector corresponds to one time phase is used. When one detector is selected, a same method is used for three-time phase detection, which is suitable for a scenario of pursuing the computational efficiency.

For example, one detector is adopted, which is denoted as a first detector. The first detector adopts the classification algorithm based on the minimum window to output the normal period, the continuous abnormal period with the sudden amplitude change, the instantaneous abnormal period, or the continuous abnormal period with the gradual amplitude change in the time-phase detection result. The instantaneous abnormal period is a waveform that appears instantaneously and lasts for a short time, and cannot be detected by a conventional detection algorithm. Therefore, the first detector adopts the classification algorithm based on the minimum window, such that the normal period, the continuous abnormal period with the sudden amplitude change, the instantaneous abnormal period, and the continuous abnormal period with the gradual amplitude change in the online signal can be accurately detected and identified. When one detector is adopted to simultaneously detect four types of time phases, the computational amount can be reduced, thereby improving detection efficiency.

For example, at least two detectors are adopted. When time-phase detection results of the detectors are all abnormal periods, the time phase of the online signal is determined based on severity grades or occurrence frequencies of the abnormal periods. The severity grades of the abnormal periods are as follows: a severity grade of the continuous abnormal period with the sudden amplitude change > a severity grade of the continuous abnormal period with the gradual amplitude change > a severity grade of the instantaneous abnormal period. When the time-phase detection results of the detectors include both the normal period and the abnormal period, a specified first logical operation is selected to determine the time phase of the online signal. The first logical operation includes any one of an "OR" operation, an "AND" operation, a "NOR" operation, and a "NAND" operation, and the first logical operation is specified based on a collection region where the signal is located.

In other words, when time-phase detection results of different detectors are all abnormal periods, the time phase of the online signal is determined based on the severity grade or occurrence frequency of the abnormal period, which can prevent missed detection and improve accuracy of closed-loop regulation and control. When the time-phase detection results of the different detectors include the normal period and the abnormal period, a final detection result of the online signal needs to be determined based on a collection region where the online signal is located. The collection region where the signal is located includes a lesion region and a non-lesion region. If the collection region of the signal is in the non-lesion region, when the detection results of the detectors include both the normal period and the abnormal period, the first logical operation may be the "AND" or "NOR" operation. For example, if 0 is output in the normal period and 1 is output in the abnormal period, a result of the "AND" operation is 0, a result of the "NOR" operation is 0, and a finally output time-phase detection result is the normal period. In the non-lesion region, a waveform characteristic of the continuous abnormal period with the sudden amplitude change, the continuous abnormal period with the gradual amplitude change, or the instantaneous abnormal period is generally not produced. Therefore, if the detector detects the abnormal period in the non-lesion region, the abnormal period is generally an artifact, and no closed-loop regulation and control is required. Therefore, an impact of the artifact needs to be excluded when the abnormal period is detected in the non-lesion region. If the collection region of the signal is located in the lesion region, when the detection results of the detectors include both the normal period and the abnormal period, the first logical operation may be the "OR" or "NAND" operation. For example, if 0 is output in the normal period and 1 is output in the abnormal period, a result of the "OR" operation is 1, a result of the "NAND" operation is 1, and the finally output time-phase detection result is the abnormal period. This can prevent the missed detection and improve the accuracy of the closed-loop regulation and control.

Specifically, when there are two detectors, which are respectively denoted as a first detector and a second detector, the first detector adopts the classification algorithm based on the minimum window to output the normal period, the continuous abnormal period with the sudden amplitude change, or the instantaneous abnormal period in the time-phase detection result. The second detector adopts the classification algorithm based on the minimum window or the early-warning algorithm based on the zero-crossing coefficient to output the normal period or the continuous abnormal period with the gradual amplitude change in the time-phase detection result. In other words, when there are two detectors, a same detection algorithm or different detection algorithms may be adopted to detect different time phases. For example, the first detector uses the classification algorithm based on the minimum window to pursue the detection time resolution for the instantaneous abnormal period and the continuous abnormal period with the sudden amplitude change, and the second detector uses the early-warning algorithm based on the zero-crossing coefficient to pursue the detection specificity for the continuous abnormal period with the gradual amplitude change. In this way, both the detection accuracy of the time phases and the high computational efficiency can be achieved.

For example, there are at least three detectors, namely a first detector, a second detector, and a third detector. The first detector adopts the classification algorithm based on the minimum window to output the normal period or the continuous abnormal period with the sudden amplitude change in the time-phase detection result. The second detector adopts the classification algorithm based on the minimum window to output the normal period or the instantaneous abnormal period in the time-phase detection result. The third detector adopts the classification algorithm based on the minimum window or the early-warning algorithm based on the zero-crossing coefficient to output the normal period or the continuous abnormal period with the gradual amplitude change in the time-phase detection result.

Specifically, the classification algorithm based on the minimum window in the present disclosure is to divide the online signal into a plurality of minimum window segments, and calculate signal eigenvalues of the minimum window segments to obtain a detection result of a single minimum window segment. Detection results of N minimum window segments are selected to obtain a determining result of a detection window segment. The time phase of the online signal is detected based on a determining result of at least one detection window segment. A window width of the minimum window segment is less than or equal to duration of an instantaneous abnormal period in an offline signal, and a window width of the detection window segment is greater than duration of the instantaneous abnormal period in the offline signal.

It can be understood that the online signal in the present disclosure is a physiological signal, namely an EEG signal. One segment of the online signal may contain different time phases, and time phases of various signals may also have different presentation forms, time-phase determining characteristics, and thresholds. However, a principle or steps of a method for detecting the online signal based on the minimum window can be shared or adaptively adjusted. For the offline signal, the continuous abnormal period with the sudden amplitude change has a significantly different waveform characteristic from the normal period, and has longer duration. Therefore, the continuous abnormal period with the sudden amplitude change is marked first. Subsequently, during a period from start time of each continuous abnormal period with the sudden amplitude change to end time of a previous continuous abnormal period with the sudden amplitude change, the instantaneous abnormal period is marked. Finally, a remaining time period is marked as the normal period. The marking is completed manually. Both the continuous abnormal period with the sudden amplitude change and the instantaneous abnormal period belong to abnormal time phases. In the present disclosure, the window width of the minimum window segment is less than or equal to the duration of the instantaneous abnormal period in the offline signal, and the window width of the detection window segment is greater than the duration of the instantaneous abnormal period in the offline signal. It can be understood that the window width of the minimum window segment and the window width of the detection window segment are obtained based on the offline signal, and the window width is duration of the segment. When the window width of the minimum window segment is less than or equal to the duration of the instantaneous abnormal period in the offline signal, sufficient time resolution can be ensured to identify a single instantaneous abnormal period. The detection window segment is composed of a plurality of minimum window segments. Therefore, the window width of the detection window segment is greater than the duration of the instantaneous abnormal period in the offline signal. Therefore, the detection method in the present disclosure can accurately distinguish the continuous abnormal period with the sudden amplitude change, the instantaneous abnormal period, and the normal period in the online signal by using a stepwise detection method of "minimum window segment → detection window segment → online signal", thereby improving accuracy of signal detection and reducing a probability of missed detection of an abnormal signal. Moreover, a method of "detecting the minimum window and determining a detection window" can also reduce the computational amount and improve the detection efficiency. Due to short duration of the instantaneous abnormal period and the relatively long duration of the continuous abnormal period with the sudden amplitude change, in order to ensure accuracy of determining two types of abnormal signals, the window width of the detection window segment is greater than the duration of the instantaneous abnormal period in the offline signal and less than or equal to the duration of the continuous abnormal period with the sudden amplitude change in the offline signal.

In the offline signal, the duration of the continuous abnormal period with the sudden amplitude change and the duration of the instantaneous abnormal period are generally selected based on design requirements, which may be average, maximum, or minimum duration of a corresponding time phase of a historical offline signal. The duration of the continuous abnormal period with the sudden amplitude change and the duration of the instantaneous abnormal period vary for different types of offline signals. For example, for the EEG signal, the duration of the continuous abnormal period with the sudden amplitude change is generally 5 seconds to 300 seconds, and the duration of the instantaneous abnormal period is generally 0.01 seconds to 0.2 seconds. Therefore, a window width of a minimum window segment of the EEG signal is ≤ 0.5 seconds, and preferably is ≤ 0.2 seconds, which may be 0.16 seconds, 0.1 seconds, 0.06 seconds, 0.03 seconds, 0.01 seconds, 0.005 seconds, 0.001 seconds, or the like. A window width of a detection window segment of the EEG signal is generally 0.2 seconds to 300 seconds, which is preferably 0.5 seconds, 1 second, 2 seconds, 6 seconds, 10 seconds, 30 seconds, 60 seconds, 120 seconds, 200 seconds, or 240 seconds. For another example, the ECG signal, the EMG signal, and the EOE signal only have the instantaneous abnormal period. In this case, the minimum window segment completely overlaps with the detection window segment. Specifically, an instantaneous abnormal period in the ECG signal is generally 0.01 seconds to 0.3 seconds. Therefore, a window width of a minimum window segment of the ECG signal is ≤ 0.5 seconds, and preferably is ≤ 0.3 seconds, which may be 0.26 seconds, 0.22 seconds, 0.16 seconds, 0.1 seconds, 0.06 seconds, 0.03 seconds, 0.01 seconds, or the like. An instantaneous abnormal period in the EMG signal is generally 0.001 seconds to 0.1 seconds. Therefore, a window width of a minimum window segment of the EMG signal is ≤ 0.5 seconds, and preferably is ≤ 0.1 seconds, which may be 0.08 seconds, 0.06 seconds, 0.03 seconds, 0.01 seconds, 0.006 seconds, 0.003 seconds, 0.001 seconds, or the like. An instantaneous abnormal period in the EOG signal is generally 0.005 seconds to 0.2 seconds. Therefore, a window width of a minimum window segment of the EOG signal is ≤ 0.5 seconds, and preferably is ≤ 0.2 seconds, which may be 0.16 seconds, 0.12 seconds, 0.1 seconds, 0.06 seconds, 0.03 seconds, 0.01 seconds, 0.006 seconds, or the like. A quantity N of minimum window segments can be adaptively adjusted based on the window width of the minimum window segment and the window width of the detection window segment.

Due to limited space here, the EEG signal is used in the solutions. The obtaining the detection result of the single minimum window segment includes: A time phase of the offline signal is marked; a threshold indicator corresponding to at least one time phase is obtained; and the single minimum window segment is classified, that is, the single minimum window segment in the online signal is compared with a threshold indicator corresponding to any one time phase, to classify the single minimum window segment as a normal minimum window or an abnormal minimum window. The threshold indicator includes at least one of a signal feature threshold and a feature difference threshold.

In other words, threshold indicators corresponding to different time phases are first obtained based on the offline signal, and then the threshold indicators are used to detect and classify each minimum window segment of the online signal. For example, a signal eigenvalue of each minimum window segment is calculated, and is compared with the signal feature threshold. If the calculated signal eigenvalue is greater than the signal feature threshold, the minimum window segment is marked as the abnormal minimum window. Otherwise, the minimum window segment is marked as the normal minimum window. Corresponding signal feature thresholds and feature difference thresholds of different time phases may be different.

For example, there may be three methods for classifying the single minimum window segment: (1) A signal eigenvalue of the single minimum window segment in the online signal is compared with the signal feature threshold, and the single minimum window segment is classified as the abnormal minimum window when the signal eigenvalue of the single minimum window segment is greater than or equal to the signal feature threshold. (2) A signal feature difference of the single minimum window segment is compared with the feature difference threshold, and the single minimum window segment is classified as the abnormal minimum window when the signal feature difference of the single minimum window segment is greater than or equal to the feature difference threshold. (3) The signal eigenvalue and the signal feature difference of the single minimum window segment are respectively compared with the signal feature threshold and the feature difference threshold, and the single minimum window segment is classified as the abnormal minimum window when the signal eigenvalue is greater than or equal to the signal feature threshold or the signal feature difference is greater than or equal to the feature difference threshold.

In other words, the signal feature threshold, the feature difference threshold, or both the signal feature threshold and the feature difference threshold may be used to classify the single minimum window segment. When both the signal feature threshold and the feature difference threshold are used, the signal eigenvalue and the signal feature difference can be compared with their corresponding thresholds. When at least one of two comparison results indicates that the minimum window segment is classified as the abnormal minimum window, the minimum window segment is classified as the abnormal minimum window. The "feature" mentioned in the present disclosure may be a line length, the zero-crossing coefficient, or the like.

For example, in order to ensure that the signal feature difference and the signal eigenvalue have a same dimension (namely N), the signal feature difference of the single minimum window segment can be calculated in the following three manners:
(1) A signal feature difference of a j^{th} minimum window segment is equal to |a signal eigenvalue of a (j+1)^{th} minimum window segment - a signal eigenvalue of the j^{th} minimum window segment|, where j=1, 2, ..., and N-1; a signal feature difference of an N^{th} minimum window segment=|a signal eigenvalue of a 1^{st} minimum window segment - a signal eigenvalue of the N^{th} minimum window segment|.
(2) A signal feature difference of a j^{th} minimum window segment is equal to |a signal eigenvalue of a (j+1)^{th} minimum window segment - a signal eigenvalue of the j^{th} minimum window segment|, where j=1, 2, ..., and N-1; a signal feature difference of an N^{th} minimum window segment=|a signal eigenvalue of the N^{th} minimum window segment - an average value of signal eigenvalues of the N minimum window segments|.
(3) A signal feature difference of a j^{th} minimum window segment is equal to |a signal eigenvalue of the j^{th} minimum window segment - a signal eigenvalue of a (j-1)^{th} minimum window segment|, where j=2, 3, ..., and N; a signal feature difference of a 1^{st} minimum window segment=|a signal eigenvalue of the 1^{st} minimum window segment - a median of signal eigenvalues of the N^{th} minimum window segments|.

In the present disclosure, the obtaining the threshold indicator corresponding to the at least one time phase includes: Based on an offline signal marked as the normal period, a change curve P1 of a quantity proportion of detected minimum window segments of the offline signal under different signal feature thresholds or feature difference thresholds is obtained. Based on an offline signal marked as the "instantaneous abnormal period", a change curve P2 of a quantity proportion of detected minimum window segments of the offline signal under the different signal feature thresholds or feature difference thresholds is obtained. Based on an offline signal marked as the "continuous abnormal period with the sudden amplitude change", a change curve P3 of a quantity proportion of detected minimum window segments of the offline signal under the different signal feature thresholds or feature difference thresholds is obtained. Based on an offline signal marked as the "continuous abnormal period with the gradual amplitude change", a change curve P4 of a quantity proportion of detected minimum window segments of the offline signal under the different signal feature thresholds or feature difference thresholds is obtained.

Corresponding feature thresholds of different time phases are obtained based on the change curve P1, a difference curve of the change curves P2 and P1, a difference curve of the change curves P3 and P1, and a difference curve of the change curves P4 and P1. A threshold indicator corresponding to the instantaneous abnormal period is a specified value of the change curve P1, a maximum difference of a P2-P1 curve, or a maximum difference of a P2÷P1 curve. A threshold indicator corresponding to the continuous abnormal period with the sudden amplitude change is the specified value of the change curve P1, a maximum difference of a P3-P1 curve, or a maximum difference of a P3÷P1 curve. A threshold indicator corresponding to the continuous abnormal period with the gradual amplitude change is the specified value of the change curve P1, a maximum difference of a P4-P1 curve, or a maximum difference of a P4÷P1 curve.

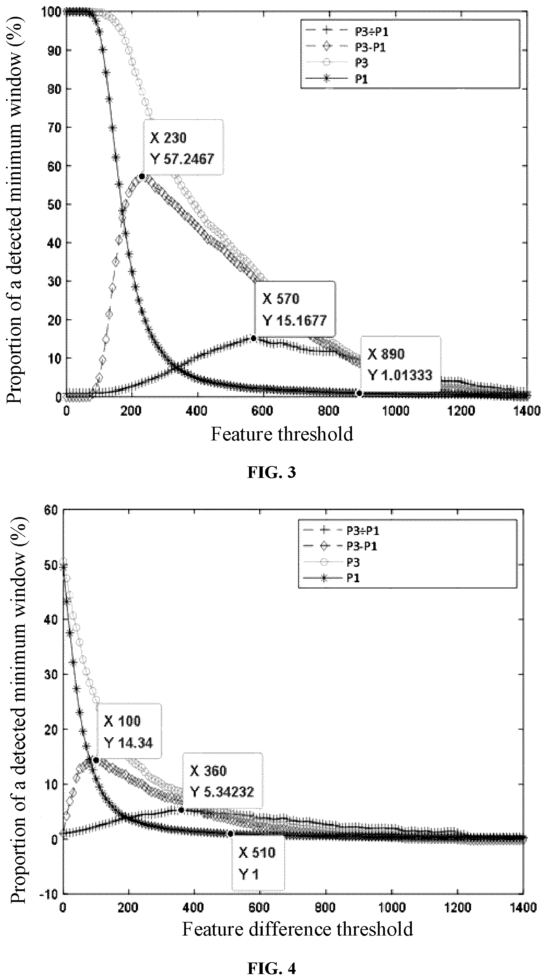

For example, the threshold indicator corresponding to the continuous abnormal period with the sudden amplitude change is taken as an example. FIG. 3 is a curve chart showing that the signal feature threshold changes with the proportion of the detected minimum window segments. Based on the change curve P1, the signal feature threshold may be the specified value 890 of the change curve P1 (under this specified value, the proportion of the detected minimum window segments in the normal period is 1%). Alternatively, the change curve P3 is subtracted from the change curve P1 to obtain the P3-P1 curve. When the signal feature threshold is 230, there is a maximum difference between the change curve P3 and the change curve P1. Alternatively, the change curve P3 is divided by the change curve P1 to obtain the P3 ÷ P1 curve. When the signal feature threshold is 570, there is a maximum difference between the change curve P3 and the change curve P1. Therefore, the signal feature threshold corresponding to the continuous abnormal period with the sudden amplitude change may be 890, 230, or 570. FIG. 4 is a curve chart showing that the feature difference threshold changes with the proportion of the detected minimum window segments. Based on the change curve P1, the feature difference threshold may be a specified value 510 of the change curve P1 (under this specified value, the proportion of the detected minimum window segments in the normal period is 1%). Alternatively, the change curve P3 is subtracted from the change curve P1 to obtain the P3-P1 curve. When the feature difference threshold is 100, there is a maximum difference between the change curve P3 and the change curve P1. Alternatively, the change curve P3 is divided by the change curve P1 to obtain the P3 ÷ P1 curve. When the feature difference threshold is 360, there is a maximum difference between the change curve P3 and the change curve P1. Therefore, the signal difference threshold corresponding to the continuous abnormal period with the sudden amplitude change may be 510, 100, or 360.

In the present disclosure, the selecting the detection results of the N minimum window segments to obtain the determining result of the detection window segment includes: Statistics is taken on a quantity of abnormal minimum window segments detected within the detection window segment, and numerical relationships between the quantity of abnormal minimum window segments detected and each of M₁, M₂, and the N is obtained by using a classification method, to obtain the determining result of the detection window segment. The M₁ is a threshold for a quantity of minimum windows obtained through traversal and selection in the continuous abnormal period with the sudden amplitude change, and the M₂ is a threshold for a quantity of minimum windows obtained through the traversal and selection in the instantaneous abnormal period.

Specifically, a method for the traversal and selection includes: Traversal is performed within a range of (0, N) to obtain a value of the threshold for the quantity of minimum windows; a detection performance indicator of the threshold for the quantity of minimum windows under each value is separately calculated; and the threshold M₁ for the quantity of minimum windows in the continuous abnormal period with the sudden amplitude change and the threshold M₂ for the quantity of minimum windows in the instantaneous abnormal period are selected based on the detection performance indicator. The detection performance indicator includes at least one of a false detection rate and a detection delay. For example, the false detection rate is less than 1 time per hour, and the detection delay is less than 12 seconds.

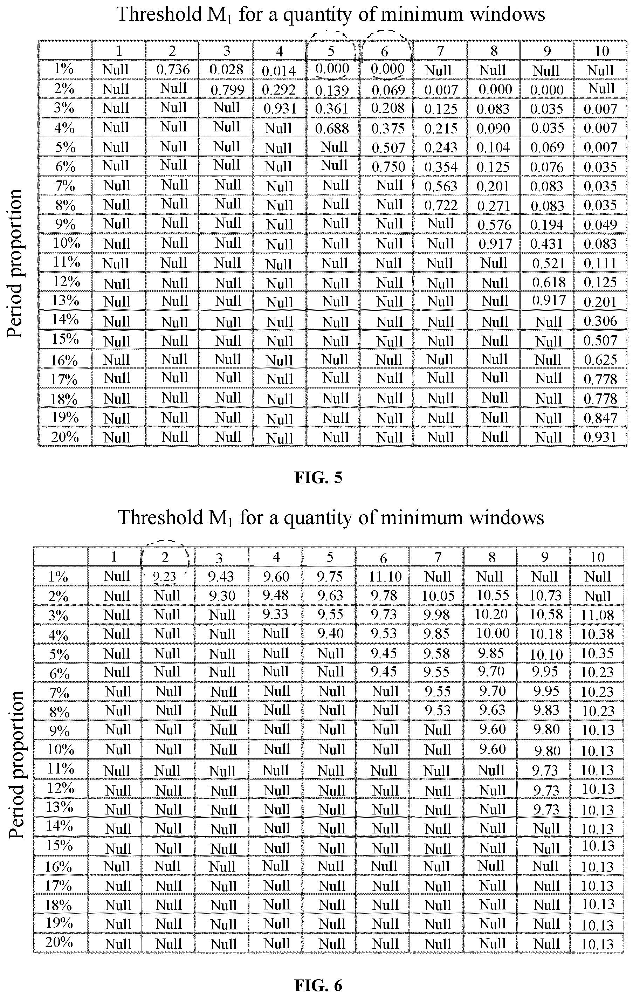

For example, taking the threshold M₁ for the quantity of minimum windows in the continuous abnormal period with the sudden amplitude change as an example, assuming that the N is set to 10, the threshold M₁ for the quantity of minimum windows is within (0,10), and the value is taken at intervals of 1, the false detection rate (as shown in FIG. 5) and the detection delay (as shown in FIG. 6) of the threshold M₁ for the quantity of minimum windows under each value are calculated. During the calculation, data that does not meet the detection performance indicators is denoted as "null", otherwise a value of the detection performance indicators is given. From the figure, it can be seen that when the threshold M₁ for the quantity of minimum windows is 5 or 6, and a proportion of the normal period in the offline signal is 1%, the false detection rate is 0%. From the figure, it can be seen that when the threshold M₁ for the quantity of minimum windows is 2, and the proportion of the normal period in the offline signal is 1%, the detection delay is shortest. In a practical application, if a lower false detection rate is pursued, the threshold M₁ for the quantity of minimum windows may be set to 5 or 6. If a shorter detection delay is pursued, the threshold M₁ for the quantity of minimum windows may be set to 2.

It should be noted that the classification method in the present disclosure includes at least one of a synchronous three-way classification method and an asynchronous three-way classification method. The synchronous three-way classification method is to perform threshold comparison once to obtain the quantity M of abnormal minimum window segments detected within the detection window segment, and compare numerical relationships between the M and each of the M₁, the M₂, and the N. When M₁≤M≤N, the determining result of the detection window segment indicates the continuous abnormal period with the sudden amplitude change. When M₂≤M<M₁, the determining result of the detection window segment indicates the instantaneous abnormal period. When 0≤M<M₂, the determining result of the detection window segment indicates the normal period. Asynchronous three-way classification is to perform the threshold comparison twice to respectively obtain quantities K₁ and K₂ of abnormal minimum window segments detected within the detection window segment (the online signal is compared based on a first threshold to distinguish between the normal minimum window and the abnormal minimum window, and the first quantity K₁ of abnormal minimum windows detected is obtained, where the first threshold is the threshold indicator corresponding to the continuous abnormal period with the sudden amplitude change; and the online signal is compared based on a second threshold to distinguish between the normal minimum window and the abnormal minimum window, and the second quantity K₂ of abnormal minimum windows detected is obtained, where the second threshold is the threshold indicator corresponding to the instantaneous abnormal period). Then numerical relationships between the K₁ and each of the M₁ and the N, as well as numerical relationships between the K₂ and each of the M₂ and the N are obtained in parallel to output the determining result of the detection window segment (the K₁ is used to determine whether the detection window segment is in the normal period or the continuous abnormal period with the sudden amplitude change; and the K₂ is used to determine whether the detection window segment is in the normal period or the instantaneous abnormal period). When different determining results are obtained for a same detection window segment, secondary distinguishing is performed through a logical operation, where priorities of the logical operation in the continuous abnormal period with the sudden amplitude change, the instantaneous abnormal period, and the normal period are in a descending order.

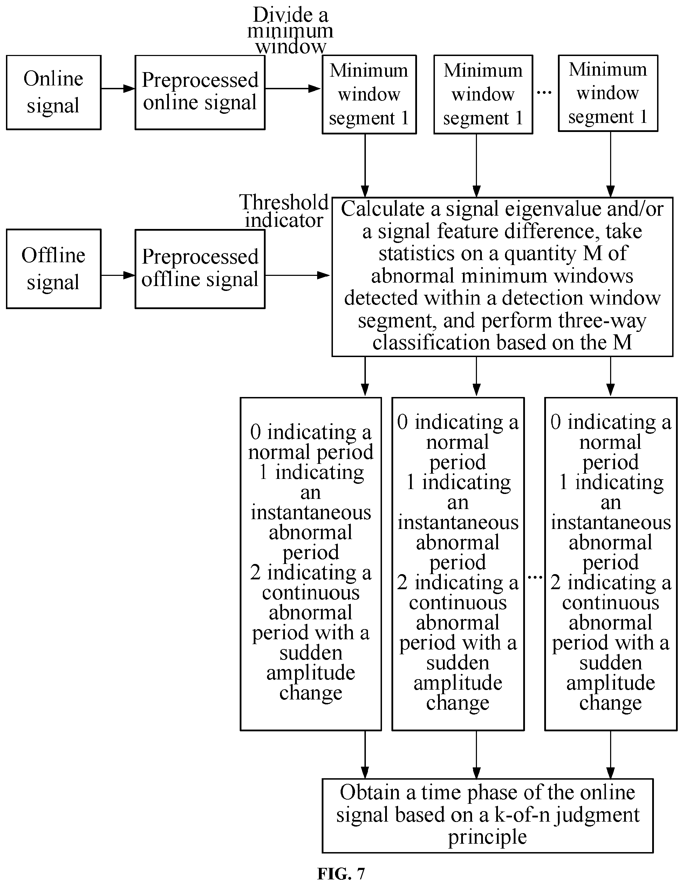

For example, as shown in FIG. 7, the online signal is divided into a plurality of minimum window segments after being preprocessed, a signal feature threshold and/or a feature difference threshold of each minimum window segment are/is calculated, and N minimum windows are selected to form a detection window segment. The signal feature threshold and/or the feature difference threshold are/is separately compared with threshold indicator (namely, determining is performed based on the first threshold) to obtain a detection result of each minimum window segment (namely, a quantity of abnormal minimum windows and a quantity of normal minimum windows). It should be noted that because different time phases have different threshold indicators, in synchronous three-way classification, in order to distinguish between the continuous abnormal period with the sudden amplitude change and the instantaneous abnormal period, a suitable threshold needs to be selected between the threshold indicator corresponding to the instantaneous abnormal period and the threshold indicator corresponding to the continuous abnormal period with the sudden amplitude change for the specified threshold indicator (this threshold can balance a quantity of detected instantaneous abnormal periods and a quantity of detected continuous abnormal periods with the sudden amplitude change). If a quantity of abnormal minimum windows within the detection window segment is M, then the M is compared with the M₁, the M₂, and the N simultaneously, and a determining result of the detection window segment is output, for example, 0 indicates the normal period, 1 indicates the instantaneous abnormal period, and 2 indicates the continuous abnormal period with the sudden amplitude change. In other words, in the synchronous three-way classification, the threshold comparison is performed only once on each minimum window segment to obtain the quantity M of abnormal minimum windows, and then three-way classification is performed on the detection window segment based on a value of the M.

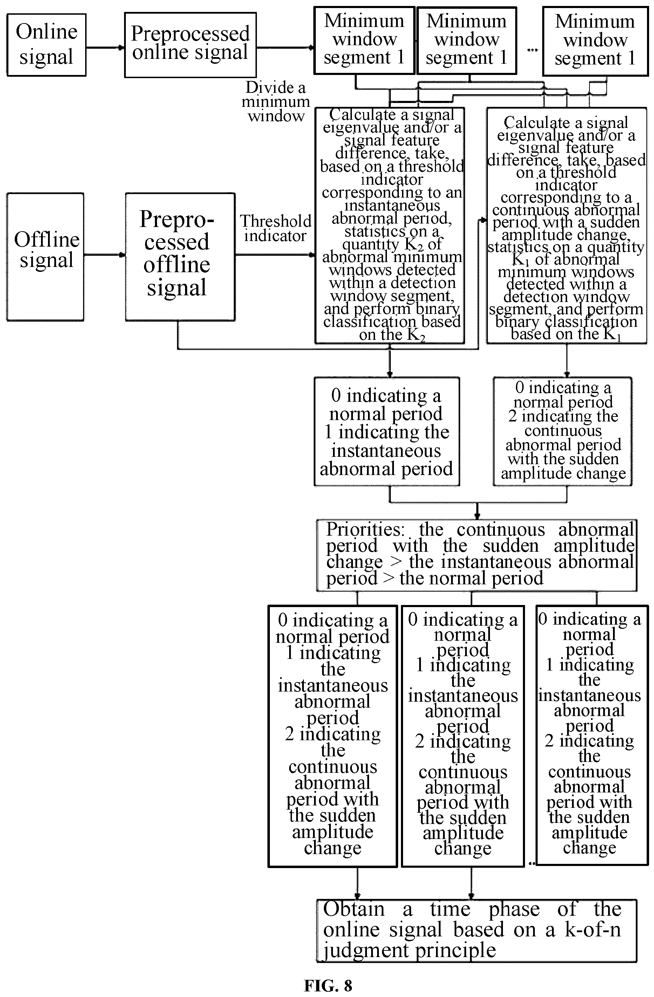

For example, as shown in FIG. 8, the online signal is divided into a plurality of minimum window segments after being preprocessed, a signal feature threshold and/or a feature difference threshold of each minimum window segment are/is calculated, and N minimum windows are selected to form a detection window segment. The signal feature threshold and/or the feature difference threshold of each minimum window segment are/is compared with the threshold indicator corresponding to the continuous abnormal period with the sudden amplitude change to obtain a detection result of each minimum window segment, and the quantity K₁ of abnormal minimum windows detected is obtained through statistics. The signal feature threshold and/or the feature difference threshold of each minimum window segment are/is compared with the threshold indicator corresponding to the instantaneous abnormal period to obtain a detection result of each minimum window segment, and the quantity K₂ of abnormal minimum windows detected is obtained through the statistics. The K₁ is compared with the M₁ and the N to obtain a binary classification result of the detection window segment (that is, 0 indicates the normal period, and 2 indicates the continuous abnormal period with the sudden amplitude change). The K₂ is compared with the M₂ and the N to obtain a binary classification result of the detection window segment (that is, 0 indicates the normal period, and 1 indicates the instantaneous abnormal period). Then, the results of the two classification operations are further distinguished through the logical operation to obtain a final determining result of the detection window segment. The priorities of the logical operation in the continuous abnormal period with the sudden amplitude change, the instantaneous abnormal period, and the normal period are in the descending order. For example, if the classification result obtained for the detection window segment by comparing the K₁ with the M₁ and the N is 0, and the classification result obtained for the detection window segment by comparing the K₂ with the M₂ and the N is 1, the output final determining result of the detection window segment is 1. For example, if the classification result obtained for the detection window segment by comparing the K₁ with the M₁ and the N is 2, and the classification result obtained for the detection window segment by comparing the K₂ with the M₂ and the N is 1, the output final determining result of the detection window segment is 2. Based on a priority order of the logical operation, the missed detection can be avoided. In other words, in the asynchronous three-way classification, the threshold determining is performed twice for the minimum window segment, and then the logical operation is performed on the results of the two binary classification operations to obtain the final determining result of the detection window segment.

As mentioned earlier, in the synchronous three-way classification method for the detection window segment, the threshold comparison only needs to be performed once for the minimum window to complete three-way classification of the detection window segment. In contrast, in the asynchronous three-way classification method, the threshold comparison needs to be performed twice for the minimum window, resulting in high computational complexity. However, a stronger capability to distinguish between the instantaneous abnormal period and the continuous abnormal period with the sudden amplitude change is achieved. The former is suitable for an application scenario of pursuing computational efficiency, a low power consumption, and high real-time performance, while the latter is suitable for an application scenario that requires high detection accuracy.

In the present disclosure, the detecting the time phase of the online signal based on the determining result of the at least one detection window segment includes: After determining results of a plurality of detection window segments are obtained, determining is performed based on a k-of-n judgment principle. In a scenario of pursuing detection specificity, the k-of-n judgment principle requires that k detection window segments are detected continuously. In a scenario of pursuing detection sensitivity, the k-of-n judgment principle requires that the k detection window segments are detected discontinuously. n represents a quantity of detection window segments contained in the online signal, and k represents a quantity of detection window segments detected among the n detection window segments.

In other words, the determining result of each detection window segment can be obtained through the synchronous or asynchronous three-way classification. One segment of the online signal can contain a plurality of detection window segments, and each detection window segment contains a plurality of minimum window segments. After the detection window segments are obtained, the determining is performed based on the k-of-n judgment principle to obtain the time phase of the online signal. This can further improve accuracy of the detection result.

For example, the N is set to 10, the M₁ is set to 8, the M₂ is set to 2, and a 1-of-1 judgment principle is followed. A criterion for the synchronous three-way classification is as follows: When 8≤M≤10, the determining result of the detection window segment indicates the continuous abnormal period with the sudden amplitude change. When 2≤M<8, the determining result of the detection window segment indicates the instantaneous abnormal period. When 0≤M<2, the determining result of the detection window segment indicates the normal period. A criterion for the asynchronous three-way classification is as follows: When 8≤K₁≤10, the determining result of the detection window segment indicates the continuous abnormal period with the sudden amplitude change. When K₁<8, the determining result of the detection window segment indicates normal period. When 2≤K₂≤10, the determining result of the detection window segment indicates the instantaneous abnormal period. When K₂<2, the determining result of the detection window segment indicates the normal period.

### Embodiment 1

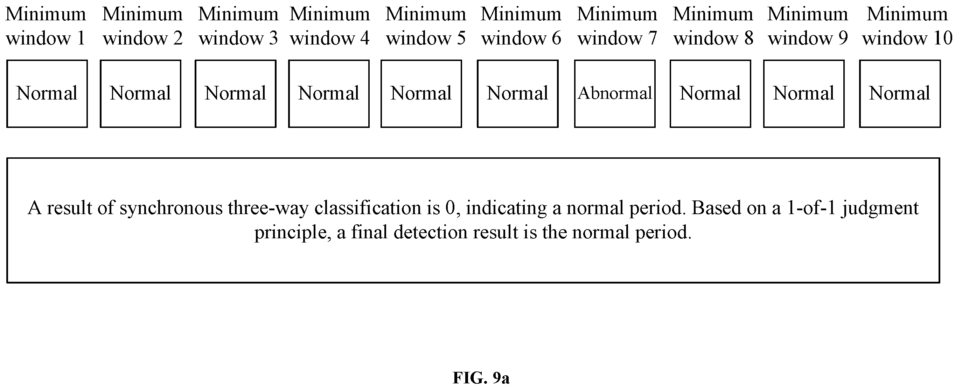

As shown in FIG. 9a, there is one abnormal minimum window among ten minimum window segments, that is, M=1. In this case, M<M₂, and the determining result of the detection window segment is 0, indicating the normal period. Based on the 1-of-1 judgment principle, the detection result of the online signal is 0, indicating the normal period.

### Embodiment 2

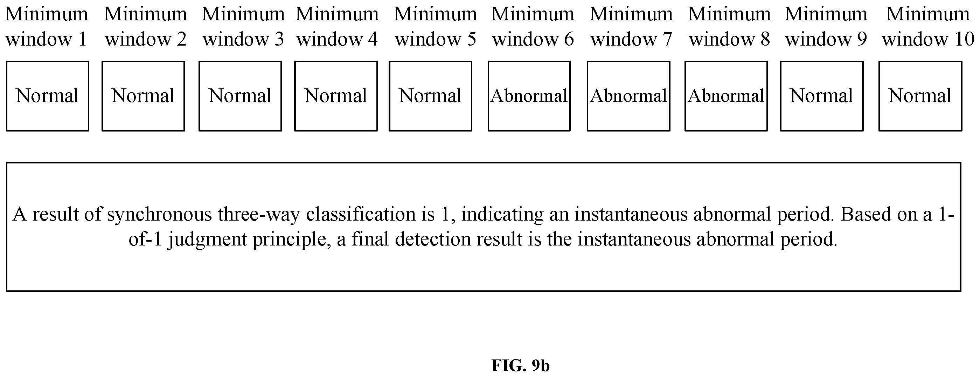

As shown in FIG. 9b, there are three abnormal minimum windows among ten minimum window segments, that is, M=3. In this case, M₂≤M<M₁, and the determining result of the detection window segment is 1, indicating the instantaneous abnormal period. Based on the 1-of-1 judgment principle, the detection result of the online signal is 1, indicating the instantaneous abnormal period.

### Embodiment 3

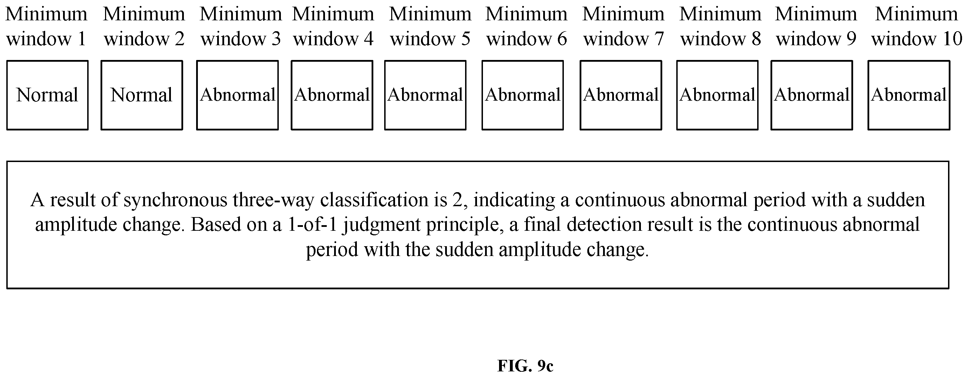

As shown in FIG. 9c, there are eight abnormal minimum windows among ten minimum window segments, that is, M=8. In this case, M₁≤M≤N, and the determining result of the detection window segment is 2, indicating the continuous abnormal period with the sudden amplitude change. Based on the 1-of-1 judgment principle, the detection result of the online signal is 2, indicating the continuous abnormal period with the sudden amplitude change.

### Embodiment 4

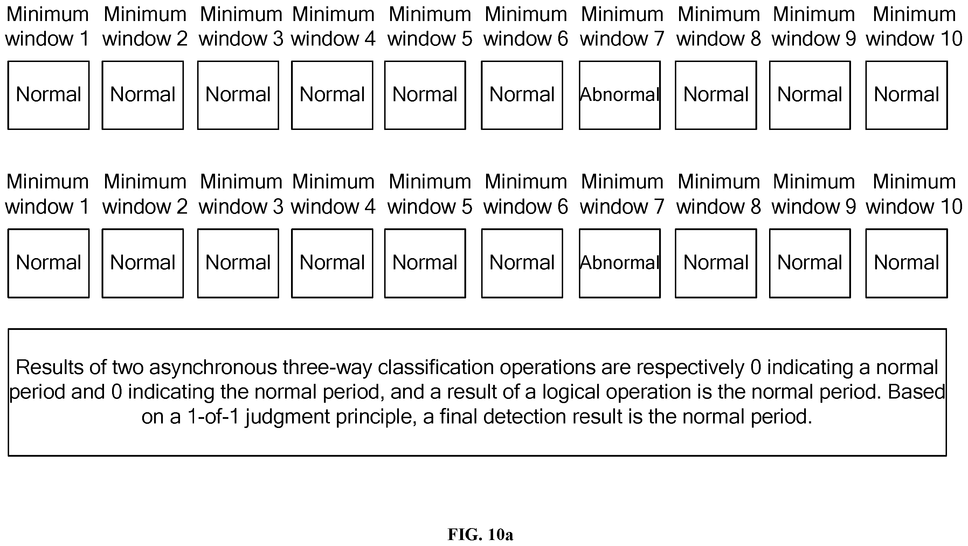

As shown in FIG. 10a, the minimum window segment is detected by using the threshold indicator corresponding to the instantaneous abnormal period, and there is one abnormal minimum window among ten minimum window segments, that is, K₂=1. In this case, K₂<M₂, and the determining result of the detection window segment is 0, indicating the normal period. The minimum window segment is detected by using the threshold indicator corresponding to the continuous abnormal period with the sudden amplitude change, and there is one abnormal minimum window among the ten minimum window segments, that is, Ki=1. In this case, K₁<M₁, and the determining result of the detection window segment is 0, indicating the normal period. The two determining results are the same. Therefore, the final determining result of the detection window segment is 0, indicating the normal period. Based on the 1-of-1 judgment principle, the detection result of the online signal is 0, indicating the normal period.

### Embodiment 5

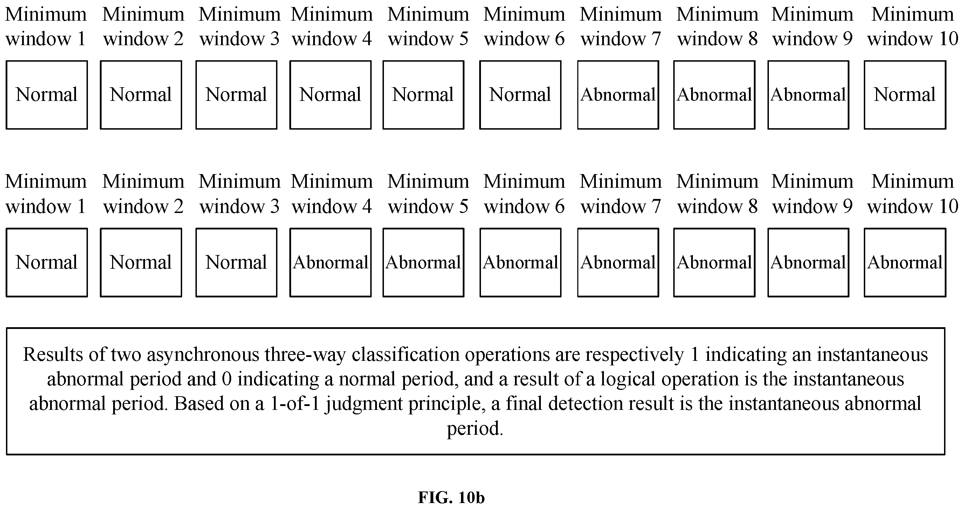

As shown in FIG. 10b, the minimum window segment is detected by using the threshold indicator corresponding to the instantaneous abnormal period, and there are three abnormal minimum windows among ten minimum window segments, that is, K₂=3. In this case, K₂>M₂, and the determining result of the detection window segment is 1, indicating the instantaneous abnormal period. The minimum window segment is detected by using the threshold indicator corresponding to the continuous abnormal period with the sudden amplitude change, and there are seven abnormal minimum windows among the ten minimum window segments, that is, K₁=7. In this case, K₁<M₁, and the determining result of the detection window segment is 0, indicating the normal period. The two determining results are different. Therefore, based on the priority, the final determining result of the detection window segment is 1, indicating the instantaneous abnormal period. Based on the 1-of-1 judgment principle, the detection result of the online signal is 1, indicating the instantaneous abnormal period.

### Embodiment 6

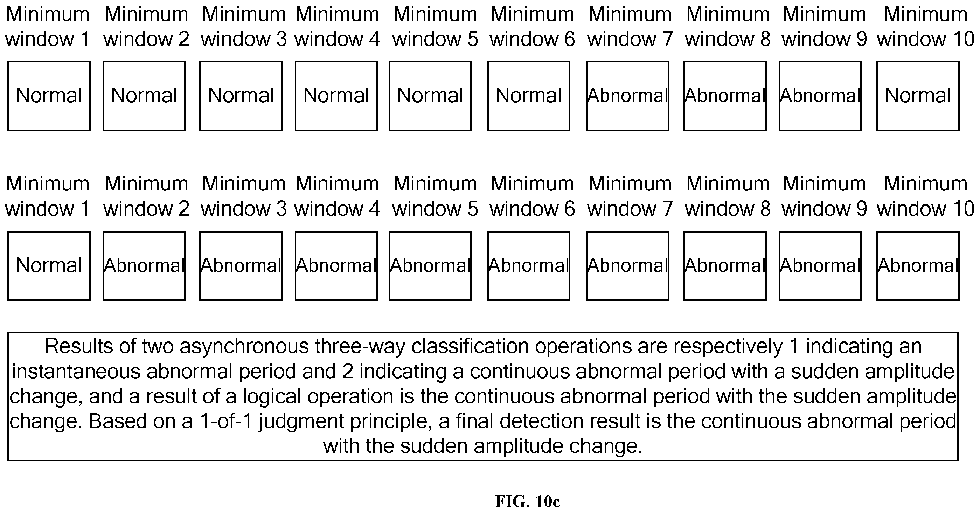

As shown in FIG. 10c, the minimum window segment is detected by using the threshold indicator corresponding to the instantaneous abnormal period, and there are three abnormal minimum windows among ten minimum window segments, that is, K₂=3. In this case, K₂>M₂, and the determining result of the detection window segment is 1, indicating the instantaneous abnormal period. The minimum window segment is detected by using the threshold indicator corresponding to the continuous abnormal period with the sudden amplitude change, and there are nine abnormal minimum windows among the ten minimum window segments, that is, Ki=9. In this case, K₁>M₁, and the determining result of the detection window segment is 2, indicating the continuous abnormal period with the sudden amplitude change. The two determining results are different. Therefore, based on the priority, the final determining result of the detection window segment is 2, indicating the continuous abnormal period with the sudden amplitude change. Based on the 1-of-1 judgment principle, the detection result of the online signal is 2, indicating the continuous abnormal period with the sudden amplitude change.

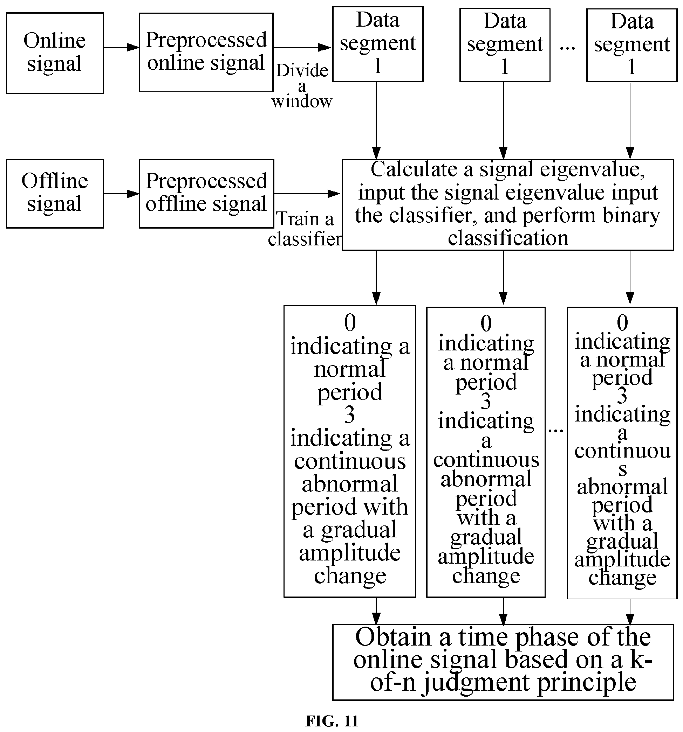

It should be noted that, as shown in FIG. 11, the early-warning algorithm based on the zero-crossing coefficient in the present disclosure is to divide the online signal into a plurality of windows to obtain a plurality of data segments; extract a signal eigenvalue for each data segment separately, and input the signal eigenvalue into a classifier to obtain a classification result of each data segment; and if classification results of X consecutive data segments are all continuous abnormal periods with the gradual amplitude change, determine that the online signal is in the continuous abnormal period with the gradual amplitude change. When at least two detectors run two different detection algorithms and the two different detection algorithms have signal eigenvalues obtained through a same linear operation, the extracted signal eigenvalue of each data segment in the early-warning algorithm based on the zero-crossing coefficient is set as a sum of the signal eigenvalue of each minimum window segment of the detection window segment in the classification algorithm based on the minimum window. In this way, a signal eigenvalue of the classification algorithm based on the minimum window is reused, such that there is no need for repeated calculation, which can reduce the computational amount and improve the detection efficiency. The signal eigenvalue includes at least one of the zero-crossing coefficient, the line length, and a curvature. It should be noted that the zero-crossing coefficient in the present disclosure is a mapping of a zero-crossing rate or a zero-crossing number. The zero-crossing coefficient is mapped into a mapping function with a positive or negative correlation, and the mapping function of the zero-crossing coefficient is linear. For example, the zero-crossing coefficient is equal to a × zero-crossing rate + b, where a is a slope of the linear mapping function, b is an intercept of the linear mapping function, and the zero-crossing rate is equal to the zero-crossing number ÷ a total quantity of sample points of the data segment. The signal eigenvalue of each data segment can be calculated to identify whether the data segment is in the normal period or the continuous abnormal period with the gradual amplitude change. In order to improve detection accuracy, it is determined that the online signal is in the continuous abnormal period with the gradual amplitude change only when the classification results of the X consecutive data segments are all the continuous abnormal periods with the gradual amplitude change. A value of the X may be set based on an actual situation, for example, may be set to 10.

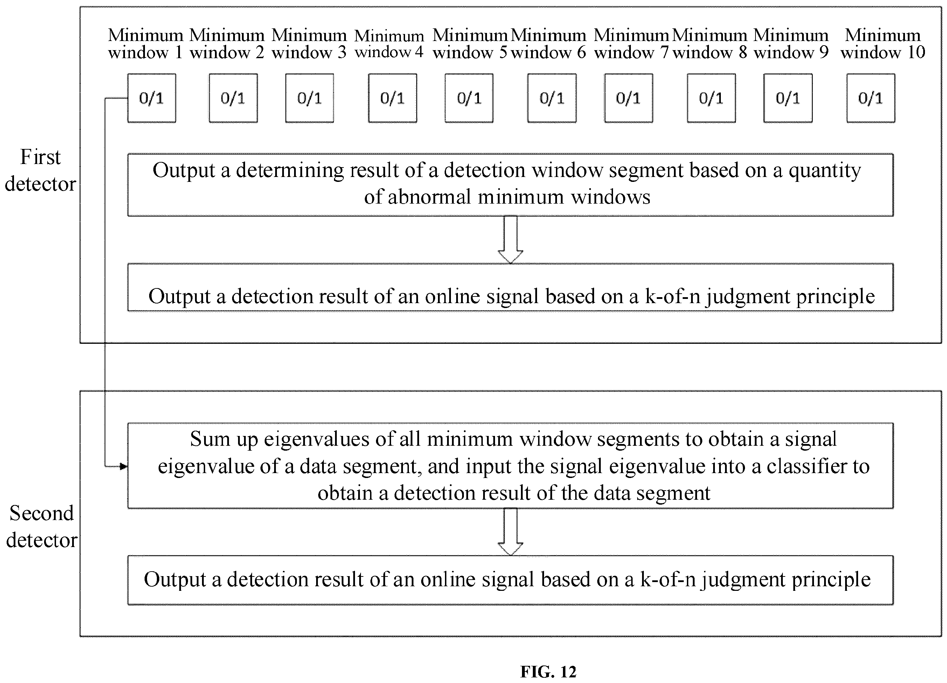

For example, as shown in FIG. 12, in an implementation solution in which two detectors are adopted, a first detector adopts the classification algorithm based on the minimum window, and a second detector adopts the early-warning algorithm based on the zero-crossing coefficient. The abnormal minimum window is denoted as 1, and the normal minimum window is denoted as 0. Based on the quantity of abnormal minimum windows, a time-phase detection result of the detection window segment is output (through the synchronous or asynchronous three-way classification). Then, based on the k-of-n judgment principle, the time-phase detection result of the online signal is obtained. The first detector is mainly configured to output the normal period, the continuous abnormal period with the sudden amplitude change, or the instantaneous abnormal period. The second detector can reuse a signal feature of the first detector, sum up signal eigenvalues of all minimum window segments within one detection window segment as a signal eigenvalue of one data segment, input the signal eigenvalue into the classifier to obtain the classification result of each data segment, and then the time-phase detection result of the online signal is obtained based on the k-of-n judgment principle. The first detector is mainly configured to output the normal period and the continuous abnormal period with the gradual amplitude change.

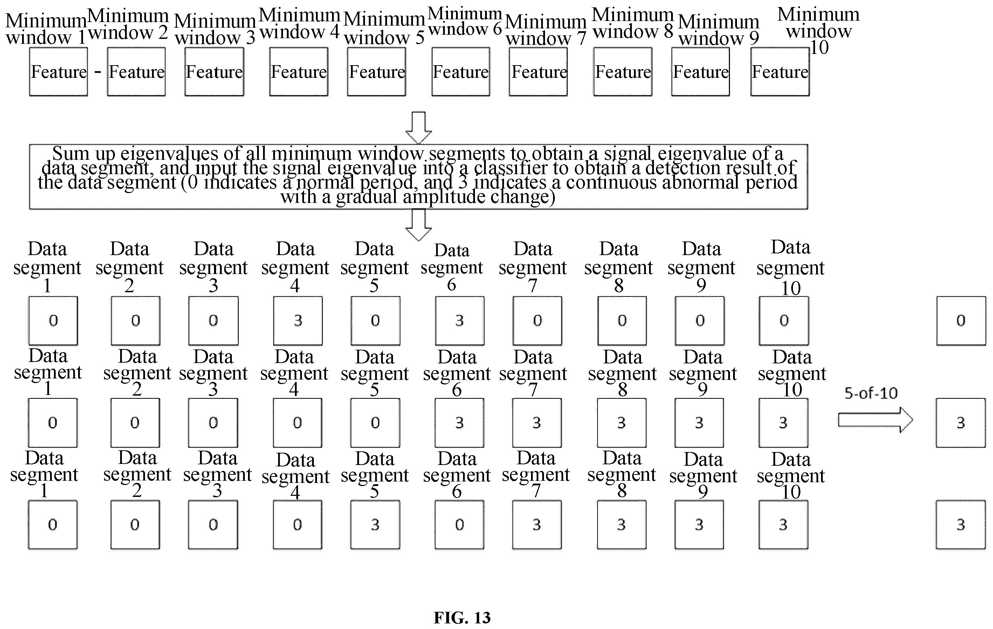

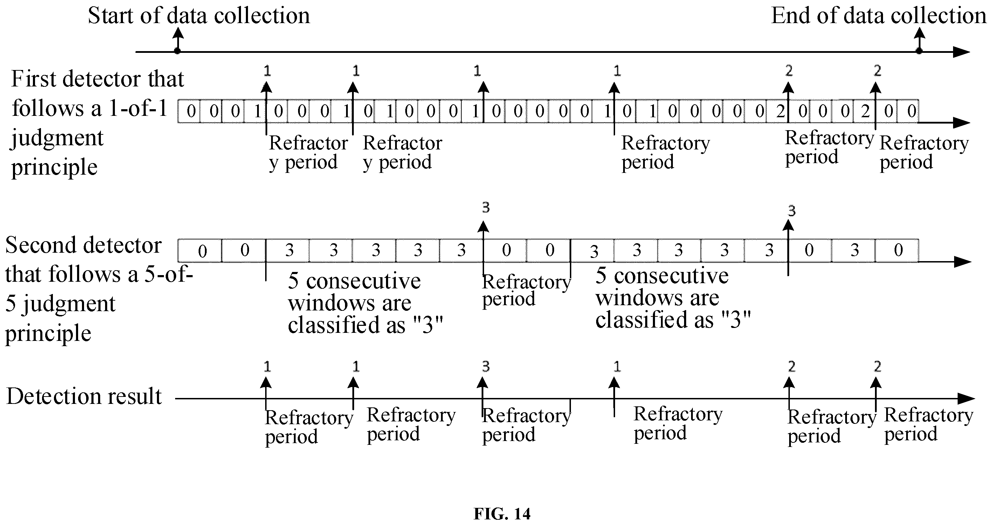

For example, for the first detector, N=5, M₁=4, M₂=1, and the 1-of-1 judgment principle is followed. For the second detector, X=5, and a 5-of-10 principle is followed. When the second detector outputs 3 indicating the continuous abnormal period with the gradual amplitude change for 5 consecutive or discontinuous data segments, it is determined that the time-phase detection result of the online signal is 3, indicating the continuous abnormal period with the gradual amplitude change (as shown in FIG. 13). When the first detector and the second detector perform detection in parallel, the output results of the first detector and the second detector can be analyzed to obtain the final detection result (as shown in FIG. 14). In the k-of-n judgment principle, values of the k and the n affect a performance orientation of the detection result. When the values of the k and the n are small, higher detection sensitivity is preferentially obtained, while when the values of the k and the n are large, the specificity is preferentially pursued. Therefore, in a practical application, k-of-n can be set by users themselves.

The present disclosure also provides a time-phase detection unit that adopts the above time-phase detection method for an online signal. The time-phase detection unit includes a detection component, which includes a plurality of detectors configured to output detection results corresponding to different time phases; a processor configured to determine a time phase of an online signal based on the detection results corresponding to the different time phases. The processor can be integrated into MCU, FPGA, CPU, and GPU chips.

The present disclosure also provides a closed-loop regulation and control system, including a time-phase detection unit, and a regulation and control module. The regulation and control module is configured to perform closed-loop regulation and control based on a time phase of an online signal.

In summary, the present disclosure adopts a plurality of detectors to simultaneously detect an online signal. Different detectors can identify different abnormal periods in a targeted manner, making a detection result more accurate and preventing missed detection. In addition, detection efficiency can also be improved.

Under the inspiration of the above ideal embodiments of the present disclosure, a skilled person can absolutely make various changes and modifications based on the above description content without departing from the scope of the technical idea of the present disclosure. The technical scope of the present disclosure is not limited to the content of this specification, which must be determined according to the scope of the claims.

## Claims

1. A time-phase detection method for an electroencephalogram signal, comprising:
using (S1) a plurality of detectors to run a detection algorithm and detect an electroencephalogram signal in parallel, wherein said plurality of detectors output time-phase detection results of the electroencephalogram signal; and
determining (S2) a time phase of the electroencephalogram signal based on each of the time-phase detection results, wherein the time-phase detection results of the electroencephalogram signal comprise an abnormal period and a normal period;
the abnormal period comprises at least one of a continuous abnormal period with a sudden amplitude change, a continuous abnormal period with a gradual amplitude change, and an instantaneous abnormal period; and the detection algorithm comprises at least one of a classification algorithm based on a minimum window and an early-warning algorithm based on a zero-crossing coefficient;
when the time-phase detection results of each of the detectors are all abnormal periods, the time phase of the electroencephalogram signal is determined based on severity grades or occurrence frequencies of the abnormal periods, wherein the severity grades of the abnormal periods are as follows: a severity grade of the continuous abnormal period with the sudden amplitude change > a severity grade of the continuous abnormal period with the gradual amplitude change > a severity grade of the instantaneous abnormal period;
when the time-phase detection results of each of the detectors comprise both the normal period and the abnormal period, a specified first logical operation is selected to determine the time phase of the electroencephalogram signal, wherein the first logical operation comprises any one of an "OR" operation, an "AND" operation, a "NOR" operation, and a "NAND" operation; and the first logical operation is specified based on a collection region where a signal is located; and
the classification algorithm based on the minimum window comprises:
dividing the electroencephalogram signal into a plurality of minimum window segments, and calculating signal eigenvalues of the plurality of minimum window segments to obtain a detection result of a single minimum window segment;
selecting detection results of N minimum window segments, wherein a determining result of a detection window segment is obtained from said selected detection results of said N minimum window segments; and
detecting the time phase of the electroencephalogram signal based on the determining result of at least one detection window segment, wherein a window width of the minimum window segments is less than or equal to a duration of an instantaneous abnormal period in an offline EEG signal, and a window width of the detection window segment is greater than the duration of the instantaneous abnormal period in the offline EEG signal.

2. The time-phase detection method for the electroencephalogram signal according to claim 1, **characterized in that**:
a number of the plurality of detectors is two, namely a first detector and a second detector are provided, wherein
the first detector adopts the classification algorithm based on the minimum window to output the normal period, the continuous abnormal period with the sudden amplitude change, or the instantaneous abnormal period in the time-phase detection result; and
the second detector adopts the classification algorithm based on the minimum window or the early-warning algorithm based on the zero-crossing coefficient to output the normal period or the continuous abnormal period with the gradual amplitude change in the time-phase detection result.

3. The time-phase detection method for the electroencephalogram signal according to claim 1, **characterized in that**:
a number of the plurality of detectors is three, namely a first detector, a second detector, and a third detector are provided, wherein
the first detector adopts the classification algorithm based on the minimum window to output the normal period or the continuous abnormal period with the sudden amplitude change in the time-phase detection result;
the second detector adopts the classification algorithm based on the minimum window to output the normal period or the instantaneous abnormal period in the time-phase detection result; and
the third detector adopts the classification algorithm based on the minimum window or the early-warning algorithm based on the zero-crossing coefficient to output the normal period or the continuous abnormal period with the gradual amplitude change in the time-phase detection result.

4. The time-phase detection method for the electroencephalogram signal according to claim 1, **characterized in that**:
the detection result of the single minimum window segment is obtained by:
marking a time phase of the offline signal;
obtaining a threshold indicator corresponding to at least one time phase; and
classifying the single minimum window segment, that is, comparing the single minimum window segment in the electroencephalogram signal with a threshold indicator corresponding to any one time phase, to classify the single minimum window segment as a normal minimum window or an abnormal minimum window, wherein the threshold indicator comprises at least one of a signal feature threshold and a feature difference threshold;
the classifying the single minimum window segment comprises:
comparing a signal eigenvalue of the single minimum window segment in the electroencephalogram signal with the signal feature threshold, and classifying the single minimum window segment as the abnormal minimum window when the signal eigenvalue of the single minimum window segment is greater than or equal to the signal feature threshold; or
comparing a signal feature difference of the single minimum window segment with the feature difference threshold, and classifying the single minimum window segment as the abnormal minimum window when the signal feature difference of the single minimum window segment is greater than or equal to the feature difference threshold; or
comparing the signal eigenvalue of the single minimum window segment with the feature threshold, and comparing the signal feature difference of the single minimum window segment with the feature difference threshold; and classifying the single minimum window segment as the abnormal minimum window when the signal eigenvalue of the single minimum window segment is greater than or equal to the signal feature threshold and/or the signal feature difference of the single minimum window segment is greater than or equal to the feature difference threshold; and
the obtaining the threshold indicator corresponding to the at least one time phase comprises:
based on an offline signal marked as the normal period, obtaining a change curve P1 of a quantity proportion of detected minimum window segments of the offline signal under different feature thresholds or feature difference thresholds;
based on an offline signal marked as the "instantaneous abnormal period", obtaining a change curve P2 of a quantity proportion of detected minimum window segments of the offline signal under the different feature thresholds or the feature difference thresholds;
based on an offline signal marked as the "continuous abnormal period with the sudden amplitude change", obtaining a change curve P3 of a quantity proportion of detected minimum window segments of the offline signal under the different feature thresholds or the feature difference thresholds;
based on an offline signal marked as the "continuous abnormal period with the gradual amplitude change", obtaining a change curve P4 of a quantity proportion of detected minimum window segments of the offline signal under the different feature thresholds or the feature difference thresholds; and
obtaining corresponding feature thresholds of different time phases based on the change curve P1, a difference curve of the change curves P2 and P1, a difference curve of the change curves P3 and P1, and a difference curve of the change curves P4 and P1, wherein
a signal feature threshold corresponding to the instantaneous abnormal period is a specified value of the change curve P1, a maximum difference of a P2-P1 curve, or a maximum difference of a P2÷P1 curve;
a signal feature threshold corresponding to the continuous abnormal period with the sudden amplitude change is the specified value of the change curve P1, a maximum difference of a P3-P1 curve, or a maximum difference of a P3÷P1 curve; and
a signal feature threshold corresponding to the continuous abnormal period with the gradual amplitude change is the specified value of the change curve P1, a maximum difference of a P4-P1 curve, or a maximum difference of a P4÷P1 curve.

5. The time-phase detection method for the electroencephalogram signal according to claim 1, **characterized in that**:
the selecting the detection results of the N minimum window segments to obtain the determining result of the detection window segment comprises:
taking statistics on a quantity of abnormal minimum window segments detected within the detection window segment; and
comparing numerical relationships between the quantity of the abnormal minimum window segments detected and each of M₁, M₂, and N by using a classification method, to obtain the determining result of the detection window segment, wherein the M₁ is a threshold for a quantity of minimum windows obtained through traversal and selection in the continuous abnormal period with the sudden amplitude change, and the M₂ is a threshold for a quantity of minimum windows obtained through the traversal and selection in the instantaneous abnormal period; wherein
a method for the traversal and selection comprises:
performing traversal within a range of (0, N) to obtain a value of the threshold for the quantity of the minimum windows;
separately calculating a detection performance indicator of the threshold for the quantity of the minimum windows under each value; and
respectively selecting, based on the detection performance indicator, the threshold M₁ for the quantity of the minimum windows in the continuous abnormal period with the sudden amplitude change and the threshold M₂ for the quantity of the minimum windows in the instantaneous abnormal period; and the detection performance indicator comprises at least one of a false detection rate and a detection delay, wherein
the false detection rate < 1 time/hour;
the detection delay < 12 seconds;
the classification method comprises at least one of a synchronous three-way classification method and an asynchronous three-way classification method;
the synchronous three-way classification method is to perform threshold comparison once to obtain the quantity M of the abnormal minimum window segments detected within the detection window segment, and compare numerical relationships between the M and each of the M₁, the M₂, and the N at the same time, wherein
when M₁≤M≤N, the determining result of the detection window segment indicates the continuous abnormal period with the sudden amplitude change;
when M₂≤M<M₁, the determining result of the detection window segment indicates the instantaneous abnormal period;
when 0≤M<M₂, the determining result of the detection window segment indicates the normal period;
the asynchronous three-way classification is to perform the threshold comparison twice to respectively obtain quantities K₁ and K₂ of the abnormal minimum window segments detected within the detection window segment, and compare numerical relationships between the Ki and each of the M₁ and the N, as well as numerical relationships between the K₂ and each of M₂ and the N in parallel; and when different determining results are obtained for a same detection window segment, a second logical operation is used for secondary differentiation, wherein priorities of the second logical operation in the continuous abnormal period with the sudden amplitude change, the instantaneous abnormal period, and the normal period are in a descending order.

6. The time-phase detection method for the electroencephalogram signal according to claim 1, **characterized in that**:
the detecting the time phase of the electroencephalogram signal based on the determining result of the at least one detection window segment comprises:
after obtaining determining results of a plurality of detection window segments, performing determining based on a k-of-n judgment principle, wherein
in a scenario of preferentially pursuing detection specificity, the k-of-n judgment principle requires that k detection window segments are detected continuously; and
in a scenario of preferentially pursuing detection sensitivity, the k-of-n judgment principle requires that the k detection window segments are detected discontinuously, wherein
n represents a quantity of detection window segments contained in the electroencephalogram signal, and k represents a quantity of detection window segments detected among n detection window segments.

7. The time-phase detection method for the electroencephalogram signal according to claim 1, **characterized in that**:
the early-warning algorithm based on the zero-crossing coefficient comprises:
dividing the electroencephalogram signal into a plurality of windows to obtain a plurality of data segments;
extracting a signal eigenvalue for each of the plurality of data segments separately, and inputting the signal eigenvalue into a classifier to obtain a classification result of each of the plurality of data segments; and
if classification results of X consecutive data segments each are the continuous abnormal period with the gradual amplitude change, determining that the electroencephalogram signal is in the continuous abnormal period with the gradual amplitude change.

8. The time-phase detection method for the electroencephalogram signal according to claim 7, **characterized in that**:
when at least two detectors run two different detection algorithms and the two different detection algorithms have signal eigenvalues obtained through a same linear operation, the extracted signal eigenvalue of each of the plurality of data segments in the early-warning algorithm based on the zero-crossing coefficient is set as a sum of signal eigenvalues of the minimum window segments of the detection window segment in the classification algorithm based on the minimum window, wherein the signal eigenvalues each comprise at least one of the zero-crossing coefficient, a line length, and a curvature.

9. A time-phase detection unit, **characterized in that** the time-phase detection unit adopts the time-phase detection method for the electroencephalogram signal according to any one of claims 1 to 8, and the time-phase detection unit comprises:
a detection component comprising a plurality of detectors configured to output detection results corresponding to different time phases; and
a processor configured to determine a time phase of an electroencephalogram signal based on the detection results corresponding to the different time phases.

10. A closed-loop regulation and control system, **characterized by** comprising:
the time-phase detection unit according to claim 9; and
a regulation and control module configured to perform closed-loop regulation and control based on a time phase of an electroencephalogram signal.

## Patentansprüche

1. Zeitphasendetektionsverfahren für ein Elektroenzephalogrammsignal, umfassend:
Verwenden (S1) einer Vielzahl von Detektoren, um einen Detektionsalgorithmus auszuführen und ein Elektroenzephalogrammsignal parallel zu detektieren, wobei die Vielzahl von Detektoren Zeitphasen-Detektionsergebnisse des Elektroenzephalogrammsignals ausgibt; und
Bestimmen (S2) einer Zeitphase des Elektroenzephalogrammsignals basierend auf jedem der Zeitphasen-Detektionsergebnisse, wobei die Zeitphasen-Detektionsergebnisse einen abnormalen Zeitraum und einen normalen Zeitraum umfassen,
wobei der abnormale Zeitraum mindestens einen von einem kontinuierlichen abnormalen Zeitraum mit einer plötzlichen Amplitudenänderung, einem kontinuierlichen abnormalen Zeitraum mit einer allmählichen Amplitudenänderung und einem momentanen abnormalen Zeitraum umfasst und der Detektionsalgorithmus mindestens einen von einem auf einem minimalen Fenster basierenden Klassifikationsalgorithmus und einem auf einem Nullkreuzkoeffizienten basierenden Frühwarnalgorithmus umfasst,
wobei die Zeitphase des Elektroenzephalogrammsignals basierend auf den Schweregraden oder den Auftrittshäufigkeiten der abnormalen Zeiträume bestimmt wird, wenn die Zeitphasen-Detektionsergebnisse jedes der Detektoren allesamt abnormale Zeiträume sind, wobei die Schweregrade der abnormalen Zeiträume wie folgt sind: der Schweregrad des kontinuierlichen abnormalen Zeitraums mit der plötzlichen Amplitudenänderung > der Schweregrad des kontinuierlichen abnormalen Zeitraums mit der allmählichen Amplitudenänderung > der Schweregrad des momentanen abnormalen Zeitraums,
wobei eine festgelegte erste logische Operation ausgewählt wird, wenn die Zeitphasen-Detektionsergebnisse jedes der Detektoren sowohl den normalen Zeitraum als auch den abnormalen Zeitraum umfassen, um die Zeitphase des Elektroenzephalogrammsignals zu bestimmen, wobei die erste logische Operation eine beliebige aus einer "OR"-Operation, einer "AND"-Operation, einer "NOR"-Operation und einer "NAND"-Operation umfasst und die erste logische Operation basierend auf einem Erfassungsbereich, in dem sich ein Signal befindet, festgelegt wird, und wobei
der auf dem minimalen Fenster basierende Klassifikationsalgorithmus umfasst:
Aufteilen des Elektroenzephalogrammsignals in eine Vielzahl von minimalen Fenstersegmenten und Berechnen von Signaleigenwerten der Vielzahl von minimalen Fenstersegmenten, um ein Detektionsergebnis eines einzelnen minimalen Fenstersegments zu erhalten;
Auswählen von Detektionsergebnissen von N minimalen Fenstersegmenten, wobei ein Bestimmungsergebnis eines Detektionsfenstersegments aus den ausgewählten Detektionsergebnissen der N minimalen Fenstersegmente erhalten wird; und
Detektieren der Zeitphase des Elektroenzephalogrammsignals basierend auf dem Bestimmungsergebnis mindestens eines Detektionsfenstersegments, wobei eine Fensterbreite der minimalen Fenstersegmente kleiner oder gleich einer Dauer eines momentanen abnormalen Zeitraums in einem Offline-EEG (Elektroenzephalogramm)-Signal ist und eine Fensterbreite des Detektionsfenstersegments größer als die Dauer des momentanen abnormalen Zeitraums in dem Offline-EEG-Signal ist.

2. Zeitphasendetektionsverfahren für ein Elektroenzephalogrammsignal nach Anspruch 1, **dadurch gekennzeichnet, dass**
die Anzahl der Vielzahl von Detektoren zwei beträgt, nämlich ein erster Detektor und ein zweiter Detektor vorgesehen sind, wobei
der erste Detektor den auf dem minimalen Fenster basierenden Klassifikationsalgorithmus anwendet, um den normalen Zeitraum, den kontinuierlichen abnormalen Zeitraum mit der plötzlichen Amplitudenänderung oder den momentanen abnormalen Zeitraum in dem Zeitphasen-Detektionsergebnis auszugeben; und
der zweite Detektor den auf dem minimalen Fenster basierenden Klassifikationsalgorithmus oder den auf dem Nullkreuzkoeffizienten basierenden Frühwarnalgorithmus anwendet, um den normalen Zeitraum oder den kontinuierlichen abnormalen Zeitraum mit der allmählichen Amplitudenänderung in dem Zeitphasen-Detektionsergebnis auszugeben.

3. Zeitphasendetektionsverfahren für ein Elektroenzephalogrammsignal nach Anspruch 1, **dadurch gekennzeichnet, dass**
die Anzahl der Vielzahl von Detektoren drei beträgt, nämlich ein erster Detektor, ein zweiter Detektor und ein dritter Detektor vorgesehen sind, wobei
der erste Detektor den auf dem minimalen Fenster basierenden Klassifikationsalgorithmus anwendet, um den normalen Zeitraum oder den kontinuierlichen abnormalen Zeitraum mit der plötzlichen Amplitudenänderung in dem Zeitphasen-Detektionsergebnis auszugeben; und
der zweite Detektor den auf dem minimalen Fenster basierenden Klassifikationsalgorithmus anwendet, um den normalen Zeitraum oder den momentanen abnormalen Zeitraum in dem Zeitphasen-Detektionsergebnis auszugeben; und
der dritte Detektor den auf dem minimalen Fenster basierenden Klassifikationsalgorithmus oder den auf dem Nullkreuzkoeffizienten basierenden Frühwarnalgorithmus anwendet, um den normalen Zeitraum oder den kontinuierlichen abnormalen Zeitraum mit der allmählichen Amplitudenänderung in dem Zeitphasen-Detektionsergebnis auszugeben.

4. Zeitphasendetektionsverfahren für ein Elektroenzephalogrammsignal nach Anspruch 1, **dadurch gekennzeichnet, dass**
das Detektionsergebnis des einzelnen minimalen Fenstersegments erhalten wird durch:
Markieren einer Zeitphase des Offline-Signals;
Erhalten eines Schwellenwertindikators, der mindestens einer Zeitphase entspricht; und
Klassifizieren des einzelnen minimalen Fenstersegments, das heißt, Vergleichen des einzelnen minimalen Fenstersegments in dem Elektroenzephalogrammsignal mit einem Schwellenwertindikator, der einer beliebigen Zeitphase entspricht, um das einzelne minimale Fenstersegment als ein normales minimales Fenster oder ein abnormales minimales Fenster zu klassifizieren, wobei der Schwellenwertindikator mindestens einen von einem Signalmerkmals-Schwellenwert und einem Merkmalsdifferenz-Schwellenwert umfasst,
wobei das Klassifizieren des einzelnen minimalen Fenstersegments umfasst:
Vergleichen eines Signaleigenwerts des einzelnen minimalen Fenstersegments in dem Elektroenzephalogrammsignal mit dem Signalmerkmals-Schwellenwert, und Klassifizieren des einzelnen minimalen Fenstersegments als das abnormale minimale Fenster, wenn der Signaleigenwert des einzelnen minimalen Fenstersegments größer oder gleich dem Signalmerkmals-Schwellenwert ist; oder
Vergleichen einer Signalmerkmalsdifferenz des einzelnen minimalen Fenstersegments mit dem Merkmalsdifferenz-Schwellenwert, und Klassifizieren des einzelnen minimalen Fenstersegments als das abnormale minimale Fenster, wenn die Signalmerkmalsdifferenz des einzelnen minimalen Fenstersegments größer oder gleich dem Merkmalsdifferenz-Schwellenwert ist; oder
Vergleichen des Signaleigenwerts des einzelnen minimalen Fenstersegments mit dem Merkmals-Schwellenwert, und Vergleichen der Signalmerkmalsdifferenz des einzelnen minimalen Fenstersegments mit dem Merkmalsdifferenz-Schwellenwert; und Klassifizieren des einzelnen minimalen Fenstersegments als das abnormale minimale Fenster, wenn der Signaleigenwert des einzelnen minimalen Fenstersegments größer oder gleich dem Signalmerkmals-Schwellenwert ist und/oder die Signalmerkmalsdifferenz des einzelnen minimalen Fenstersegments größer oder gleich dem Merkmalsdifferenz-Schwellenwert ist; und wobei
das Erhalten des Schwellenwertindikators, der der mindestens einen Zeitphase entspricht, umfasst:
basierend auf einem Offline-Signal, das als der normale Zeitraum markiert ist, Erhalten einer Änderungskurve P1 eines Mengenanteils detektierter minimaler Fenstersegmente des Offline-Signals unter verschiedenen Merkmals-Schwellenwerten oder Merkmalsdifferenz-Schwellenwerten;
basierend auf einem Offline-Signal, das als der "momentane abnormale Zeitraum" markiert ist, Erhalten einer Änderungskurve P2 eines Mengenanteils detektierter minimaler Fenstersegmente des Offline-Signals unter den verschiedenen Merkmals-Schwellenwerten oder den Merkmalsdifferenz-Schwellenwerten;
basierend auf einem Offline-Signal, das als der "kontinuierliche abnormale Zeitraum mit der plötzlichen Amplitudenänderung" markiert ist, Erhalten einer Änderungskurve P3 eines Mengenanteils detektierter minimaler Fenstersegmente des Offline-Signals unter den verschiedenen Merkmals-Schwellenwerten oder den Merkmalsdifferenz-Schwellenwerten;
basierend auf einem Offline-Signal, das als der "kontinuierliche abnormale Zeitraum mit der allmählichen Amplitudenänderung" markiert ist, Erhalten einer Änderungskurve P4 eines Mengenanteils detektierter minimaler Fenstersegmente des Offline-Signals unter den verschiedenen Merkmals-Schwellenwerten oder den Merkmalsdifferenz-Schwellenwerten; und
Erhalten entsprechender Merkmals-Schwellenwerte verschiedener Zeitphasen basierend auf der Änderungskurve P1, einer Differenzkurve der Änderungskurven P2 und P1, einer Differenzkurve der Änderungskurven P3 und P1 und einer Differenzkurve der Änderungskurven P4 und P1, wobei
ein Signalmerkmals-Schwellenwert, der dem momentanen abnormalen Zeitraum entspricht, ein festgelegter Wert der Änderungskurve P1, eine maximale Differenz einer P2**-**P1-Kurve oder eine maximale Differenz einer P2/P1-Kurve ist;
ein Signalmerkmals-Schwellenwert, der dem kontinuierlichen abnormalen Zeitraum mit der plötzlichen Amplitudenänderung entspricht, der festgelegte Wert der Änderungskurve P1, eine maximale Differenz einer P3-P1-Kurve oder eine maximale Differenz einer P3/P1-Kurve ist; und
ein Signalmerkmals-Schwellenwert, der dem kontinuierlichen abnormalen Zeitraum mit der allmählichen Amplitudenänderung entspricht, der festgelegte Wert der Änderungskurve P1, eine maximale Differenz einer P4-P1-Kurve oder eine maximale Differenz einer P4/P1-Kurve ist.

5. Zeitphasendetektionsverfahren für ein Elektroenzephalogrammsignal nach Anspruch 1, **dadurch gekennzeichnet, dass**
das Auswählen der Detektionsergebnisse der N minimalen Fenstersegmente, um das Bestimmungsergebnis des Detektionsfenstersegments zu erhalten, umfasst:
Erfassen einer Statistik über eine Anzahl von innerhalb des Detektionsfenstersegments detektierten abnormalen minimalen Fenstersegmenten; und
Vergleichen von numerischen Beziehungen zwischen der Anzahl der detektierten abnormalen minimalen Fenstersegmente und jedem von M₁, M₂ und N unter Verwendung eines Klassifikationsverfahrens, um das Bestimmungsergebnis des Detektionsfenstersegments zu erhalten, wobei M₁ ein Schwellenwert für eine Anzahl von minimalen Fenstern ist, der durch Durchlaufen und Auswählen in dem kontinuierlichen abnormalen Zeitraum mit der plötzlichen Amplitudenänderung erhalten wird, und M₂ ein Schwellenwert für eine Anzahl von minimalen Fenstern ist, der durch das Durchlaufen und Auswählen in dem momentanen abnormalen Zeitraum erhalten wird; wobei
ein Verfahren für das Durchlaufen und Auswählen umfasst:
Durchführen eines Durchlaufs innerhalb eines Bereichs von (0, N), um einen Wert des Schwellenwerts für die Anzahl der minimalen Fenster zu erhalten;
separates Berechnen eines Detektionsleistungsindikators des Schwellenwerts für die Anzahl der minimalen Fenster bei jedem Wert; und
jeweiliges Auswählen, basierend auf dem Detektionsleistungsindikator, des Schwellenwerts M₁ für die Anzahl der minimalen Fenster in dem kontinuierlichen abnormalen Zeitraum mit der plötzlichen Amplitudenänderung und des Schwellenwerts M₂ für die Anzahl der minimalen Fenster in dem momentanen abnormalen Zeitraum, wobei der Detektionsleistungsindikator mindestens einen von einer Falscherkennungsrate und einer Detektionsverzögerung umfasst, wobei
die Falscherkennungsrate < 1 Mal pro Stunde ist;
die Detektionsverzögerung < 12 Sekunden ist;
wobei das Klassifikationsverfahren mindestens eines von einem synchronen Drei-Wege-Klassifikationsverfahren und einem asynchronen Drei-Wege-Klassifikationsverfahren umfasst;
wobei das synchrone Drei-Wege-Klassifikationsverfahren darin besteht, einmalig einen Schwellenwertvergleich durchzuführen, um die Anzahl M der innerhalb des Detektionsfenstersegments detektierten abnormalen minimalen Fenstersegmente zu erhalten, und gleichzeitig numerische Beziehungen zwischen M und jedem von M₁, M₂ und N zu vergleichen, wobei
das Bestimmungsergebnis des Detektionsfenstersegments den kontinuierlichen abnormalen Zeitraum mit der plötzlichen Amplitudenänderung anzeigt, wenn M₁ ≤ M ≤ N gilt;
das Bestimmungsergebnis des Detektionsfenstersegments den momentanen abnormalen Zeitraum anzeigt, wenn M₂ ≤ M < M₁ gilt;
das Bestimmungsergebnis des Detektionsfenstersegments den normalen Zeitraum anzeigt, wenn 0 ≤ M < M₂ gilt;
wobei das asynchrone Drei-Wege-Klassifikationsverfahren darin besteht, den Schwellenwertvergleich zweimal durchzuführen, um jeweils die Anzahlen K₁ und K₂ der innerhalb des Detektionsfenstersegments detektierten abnormalen minimalen Fenstersegmente zu erhalten, und parallel numerische Beziehungen zwischen K₁ und jedem von M₁ und N sowie numerische Beziehungen zwischen K₂ und jedem von M₂ und N zu vergleichen, und wobei eine zweite logische Operation zur sekundären Unterscheidung verwendet wird, wenn für dasselbe Detektionsfenstersegment unterschiedliche Bestimmungsergebnisse erhalten werden, wobei die Prioritäten der zweiten logischen Operation in dem kontinuierlichen abnormalen Zeitraum mit der plötzlichen Amplitudenänderung, dem momentanen abnormalen Zeitraum und dem normalen Zeitraum in absteigender Reihenfolge sind.

6. Zeitphasendetektionsverfahren für ein Elektroenzephalogrammsignal nach Anspruch 1, **dadurch gekennzeichnet, dass**
das Detektieren der Zeitphase des Elektroenzephalogrammsignals basierend auf dem Bestimmungsergebnis des mindestens einen Detektionsfenstersegments umfasst:
Durchführen einer Bestimmung basierend auf einem k-aus-n-Beurteilungsprinzip nach dem Erhalten von Bestimmungsergebnissen einer Vielzahl von Detektionsfenstersegmenten, wobei
in einem Szenario, in dem bevorzugt Detektionsspezifität angestrebt wird, das k-aus-n-Beurteilungsprinzip erfordert, dass k Detektionsfenstersegmente kontinuierlich detektiert werden; und
in einem Szenario, in dem bevorzugt Detektionssensitivität angestrebt wird, das k-aus-n-Beurteilungsprinzip erfordert, dass die k Detektionsfenstersegmente diskontinuierlich detektiert werden, wobei
n eine Anzahl von Detektionsfenstersegmenten darstellt, die in dem Elektroenzephalogrammsignal enthalten sind, und k eine Anzahl von Detektionsfenstersegmenten darstellt, die unter n Detektionsfenstersegmenten detektiert werden.

7. Zeitphasendetektionsverfahren für ein Elektroenzephalogrammsignal nach Anspruch 1, **dadurch gekennzeichnet, dass**
der auf dem Nullkreuzkoeffizienten basierende Frühwarnalgorithmus umfasst:
Aufteilen des Elektroenzephalogrammsignals in eine Vielzahl von Fenstern, um eine Vielzahl von Datensegmenten zu erhalten;
separates Extrahieren eines Signaleigenwerts für jedes der Vielzahl von Datensegmenten und Eingeben des Signaleigenwerts in einen Klassifikator, um ein Klassifikationsergebnis für jedes der Vielzahl von Datensegmenten zu erhalten; und
Bestimmen, dass sich das Elektroenzephalogrammsignal in dem kontinuierlichen abnormalen Zeitraum mit der allmählichen Amplitudenänderung befindet, falls die Klassifikationsergebnisse von X aufeinanderfolgenden Datensegmenten jeweils der kontinuierliche abnormale Zeitraum mit der allmählichen Amplitudenänderung sind.

8. Zeitphasendetektionsverfahren für ein Elektroenzephalogrammsignal nach Anspruch 7, **dadurch gekennzeichnet, dass**,
wenn mindestens zwei Detektoren zwei verschiedene Detektionsalgorithmen ausführen und die zwei verschiedenen Detektionsalgorithmen Signaleigenwerte, die durch eine gleiche lineare Operation erhalten werden, aufweisen, der extrahierte Signaleigenwert jedes der Vielzahl von Datensegmenten in dem auf dem Nullkreuzkoeffizienten basierenden Frühwarnalgorithmus als eine Summe von Signaleigenwerten der minimalen Fenstersegmente des Detektionsfenstersegments in dem auf dem minimalen Fenster basierenden Klassifikationsalgorithmus festgelegt wird, wobei die Signaleigenwerte jeweils mindestens eines von dem Nullkreuzkoeffizienten, einer Linienlänge und einer Krümmung umfassen.

9. Zeitphasendetektionseinheit, **dadurch gekennzeichnet, dass** die Zeitphasendetektionseinheit ein Zeitphasendetektionsverfahren für ein Elektroenzephalogrammsignal nach einem der Ansprüche 1 bis 8 anwendet, und die Zeitphasendetektionseinheit umfasst:
eine Detektionskomponente, die eine Vielzahl von Detektoren umfasst, die dazu konfiguriert sind, verschiedenen Zeitphasen entsprechende Detektionsergebnisse auszugeben; und
einen Prozessor, der dazu konfiguriert ist, eine Zeitphase eines Elektroenzephalogrammsignals basierend auf den verschiedenen Zeitphasen entsprechenden Detektionsergebnissen zu bestimmen.

10. Geschlossenes Regelungs- und Steuerungssystem, **dadurch gekennzeichnet, dass** es umfasst:
eine Zeitphasendetektionseinheit nach Anspruch 9; und
ein Regelungs- und Steuerungsmodul, das dazu konfiguriert ist, eine Regelung und Steuerung mit geschlossenem Regelkreis basierend auf einer Zeitphase eines Elektroenzephalogrammsignals durchzuführen.

## Revendications

1. Procédé de détection de phases temporelles pour un signal d'électroencéphalogramme, comprenant :
utiliser (S1) une pluralité de détecteurs pour exécuter un algorithme de détection et détecter un signal d'électroencéphalogramme en parallèle, la pluralité de détecteurs produisant des résultats de détection de phases temporelles du signal d'électroencéphalogramme ; et
déterminer (S2) une phase temporelle du signal d'électroencéphalogramme en fonction de chacun des résultats de détection de phases temporelles, les résultats de détection de phases temporelles du signal d'électroencéphalogramme comprenant une période anormale et une période normale ;
dans lequel, la période anormale comprend au moins l'un parmi une période anormale continue avec une variation d'amplitude soudaine, une période anormale continue avec une variation d'amplitude progressive, et une période anormale instantanée ; et l'algorithme de détection comprend au moins l'un d'un algorithme de classification en fonction d'une fenêtre minimale et d'un algorithme d'alerte précoce en fonction d'un coefficient de passage par zéro ;
lorsque les résultats de détection de phases temporelles de chacun des détecteurs sont tous des périodes anormales, la phase temporelle du signal d'électroencéphalogramme est déterminée en fonction des niveaux de gravité ou des fréquences d'apparition des périodes anormales, les niveaux de gravité des périodes anormales étant les suivants : un niveau de gravité de la période anormale continue avec la variation d'amplitude soudaine > un niveau de gravité de la période anormale continue avec la variation d'amplitude progressive > un niveau de gravité de la période anormale instantanée ;
lorsque les résultats de détection de phases temporelles de chacun des détecteurs comprennent à la fois la période normale et la période anormale, une première opération logique spécifiée est sélectionnée pour déterminer la phase temporelle du signal d'électroencéphalogramme, la première opération logique comprenant l'une parmi une opération « OU », une opération « ET », une opération « NOR » et une opération « NAND » ; et la première opération logique étant spécifiée en fonction d'une région de collecte dans laquelle se trouve un signal se trouve ; et
l'algorithme de classification en fonction de la fenêtre minimale consiste à :
diviser le signal d'électroencéphalogramme en une pluralité de segments de fenêtre minimale, et calculer des valeurs propres de signal de la pluralité de segments de fenêtre minimale pour obtenir un résultat de détection d'un seul segment de fenêtre minimale ;
sélectionner des résultats de détection de N segments de fenêtre minimale, un résultat de détermination d'un segment de fenêtre de détection étant obtenu à partir des résultats de détection sélectionnés des N segments de fenêtre minimale ; et
détecter la phase temporelle du signal d'électroencéphalogramme en fonction du résultat de détermination d'au moins un segment de fenêtre de détection, une largeur de fenêtre des segments de fenêtre minimale étant inférieure ou égale à une durée d'une période anormale instantanée dans un signal EEG hors ligne, et une largeur de fenêtre du segment de fenêtre de détection est supérieure à la durée de la période anormale instantanée dans le signal EEG hors ligne.

2. Procédé de détection de phases temporelles pour un signal d'électroencéphalogramme selon la revendication 1, **caractérisé en ce que** :
un nombre des détecteurs est de deux, c'est-à-dire un premier détecteur et un deuxième détecteur sont prévus, dans lesquels
le premier détecteur adopte l'algorithme de classification en fonction de la fenêtre minimale pour produire la période normale, la période anormale continue avec la variation d'amplitude soudaine, ou la période anormale instantanée dans le résultat de détection de phases temporelles ; et
le deuxième détecteur adopte l'algorithme de classification en fonction de la fenêtre minimale ou l'algorithme d'alerte précoce en fonction du coefficient de passage par zéro pour produire la période normale ou la période anormale continue avec la variation d'amplitude progressive dans le résultat de détection de phases temporelles.

3. Procédé de détection de phases temporelles pour un signal d'électroencéphalogramme selon la revendication 1, **caractérisé en ce que** :
un nombre des détecteurs est de trois, c'est-à-dire un premier détecteur et un deuxième détecteur et un troisième détecteur sont prévus, dans lesquels
le premier détecteur adopte l'algorithme de classification en fonction de la fenêtre minimale pour produire la période normale ou la période anormale continue avec la variation d'amplitude soudaine dans le résultat de détection de phases temporelles ;
le deuxième détecteur adopte l'algorithme de classification en fonction de la fenêtre minimale pour produire la période normale ou la période anormale instantanée dans le résultat de détection de phases temporelles ; et
le troisième détecteur adopte l'algorithme de classification en fonction de la fenêtre minimale ou l'algorithme d'alerte précoce en fonction du coefficient de passage par zéro pour produire la période normale ou la période anormale continue avec la variation d'amplitude progressive dans le résultat de détection de phases temporelles.

4. Procédé de détection de phases temporelles pour un signal d'électroencéphalogramme selon la revendication 1, **caractérisé en ce que** :
le résultat de détection du seul segment de fenêtre minimale est obtenu en :
marquant une phase temporelle du signal hors ligne ;
obtenant un indicateur de seuil correspondant à au moins une phase temporelle ; et
classifiant le seul segment de fenêtre minimale, c'est-à-dire en comparant le seul segment de fenêtre minimale dans le signal d'électroencéphalogramme avec un indicateur de seuil correspondant à une phase temporelle quelconque, pour classer le seul segment de fenêtre minimale comme fenêtre minimale normale ou fenêtre minimale anormale, l'indicateur de seuil comprenant au moins l'un d'un seuil de caractéristique de signal et un seuil de différence de caractéristique ;
la classification du seul segment de fenêtre minimale consiste à :
comparer une valeur propre de signal du seul segment de fenêtre minimale dans le signal d'électroencéphalogramme avec le seuil de caractéristique de signal, et classer le seul segment de fenêtre minimale comme fenêtre minimale anormale lorsque la valeur propre de signal du seul segment de fenêtre minimale est supérieure ou égale au seuil de caractéristique de signal ; ou
comparer une différence de caractéristique de signal du seul segment de fenêtre minimale avec le seuil de différence de caractéristique, et classer le seul segment de fenêtre minimale comme fenêtre minimale anormale lorsque la différence de caractéristique de signal du seul segment de fenêtre minimale est supérieure ou égale au seuil de différence de caractéristiques ; ou
comparer la valeur propre de signal du seul segment de fenêtre minimale avec le seuil de caractéristique, comparer la différence de caractéristique de signal du seul segment de fenêtre minimale avec le seuil de différence de caractéristique ; et classer le seul segment de fenêtre minimale comme fenêtre minimale anormale lorsque la valeur propre de signal du seul segment de fenêtre minimale est supérieure ou égale au seuil de caractéristique de signal et/ou que la différence de caractéristique de signal du seul segment de fenêtre minimale est supérieure ou égale au seuil de différence de caractéristiques ; et
l'obtention de l'indicateur de seuil correspondant à au moins une phase temporelle consiste à :
en fonction d'un signal hors ligne marqué comme période normale, obtenir une courbe de variation P1 d'une proportion de quantité des segments de fenêtre minimale détectés du signal hors ligne sous des différents seuils de caractéristique ou des seuils de différence de caractéristique ;
en fonction d'un signal hors ligne marqué comme « période anormale instantanée », obtenir une courbe de variation P2 d'une proportion de quantité des segments de fenêtre minimale détectés du signal hors ligne sous les différents seuils de caractéristique ou les seuils de différence de caractéristique ;
en fonction d'un signal hors ligne marqué comme « période anormale continue avec la variation d'amplitude soudaine », obtenir une courbe de variation P3 d'une proportion de quantité des segments de fenêtre minimale détectés du signal hors ligne sous les différents seuils de caractéristique ou les seuils de différence de caractéristique ;
en fonction d'un signal hors ligne marqué comme « période anormale continue avec la variation d'amplitude progressive », obtenir une courbe de variation P4 d'une proportion de quantité des segments de fenêtre minimale détectés du signal hors ligne sous les différents seuils de caractéristique ou les seuils de différence de caractéristique ; et
obtenir des seuils de caractéristiques correspondants des différentes phases temporelles en fonction de la courbe de variation P1, d'une courbe de différence des courbes de variation P2 et P1, d'une courbe de différence des courbes de variation P3 et P1, et d'une courbe de différence des courbes de variation P4 et P1, dans lesquels :
un seuil de caractéristique de signal correspondant à la période anormale instantanée est une valeur spécifiée de la courbe de variation P1, une différence maximale de la courbe P2-P1, ou une différence maximale de la courbe P2÷P1 ;
un seuil de caractéristique de signal correspondant à la période anormale continue avec la variation d'amplitude soudaine est la valeur spécifiée de la courbe de variation P1, une différence maximale d'une courbe P3-P1, ou une différence maximale d'une courbe P3÷P1 ; et
un seuil de caractéristique de signal correspondant à la période anormale continue avec la variation d'amplitude progressive est la valeur spécifiée de la courbe de variation P1, une différence maximale d'une courbe P4-P1, ou une différence maximale d'une courbe P4÷P1.

5. Procédé de détection de phases temporelles pour un signal d'électroencéphalogramme selon la revendication 1, **caractérisé en ce que** :
la sélection des résultats de détection des N segments de fenêtre minimale pour obtenir le résultat de détermination du segment de fenêtre de détection consiste à :
prendre des statistiques sur une quantité de segments de fenêtre minimale anormaux détectés dans le segment de fenêtre de détection ; et
comparer des relations numériques entre la quantité de segments de fenêtre minimale anormaux détectés et chacun de M₁, M₂, et N en utilisant un procédé de classification, pour obtenir le résultat de détermination du segment de fenêtre de détection, M₁ étant un seuil pour une quantité de fenêtres minimales obtenues par traversée et sélection pendant la période anormale continue avec la variation d'amplitude soudaine, et M₂ étant un seuil pour une quantité de fenêtres minimales obtenues par traversée et sélection pendant la période anormale instantanée ; dans lequel
un procédé de la traversée et de la sélection comprend :
effectuer une traversée dans une plage (0, N) pour obtenir une valeur du seuil pour la quantité de fenêtres minimales ;
calculer séparément un indicateur de performance de détection du seuil pour la quantité de fenêtres minimales sous chaque valeur ; et
sélectionner respectivement, en fonction l'indicateur de performance de détection, le seuil M₁ pour la quantité de fenêtres minimales dans la période anormale continue avec la variation d'amplitude soudaine et le seuil M₂ pour la quantité de fenêtres minimales dans la période anormale instantanée ; l'indicateur de performance de détection comprenant au moins l'un d'un taux de fausse détection et d'un retard de détection, dans lequel
le taux de fausse détection < 1 fois/heure ;
le retard de détection < 12 secondes ;
le procédé de classification comprend au moins l'un d'un procédé de classification synchrone à trois voies et d'un procédé de classification asynchrone à trois voies ;
le procédé de classification synchrone à trois voies consiste à effectuer une comparaison de seuils une fois pour obtenir la quantité M des segments de fenêtre minimale anormaux détectés dans le segment de fenêtre de détection, et à comparer des relations numériques entre M et chacun de M₁, M₂ et N en même temps, dans lequel,
lorsque M₁≤M≤N, le résultat de détermination du segment de fenêtre de détection indique la période anormale continue avec la variation d'amplitude soudaine ;
lorsque M₂≤M<M₁, le résultat de détermination du segment de fenêtre de détection indique la période anormale instantanée ;
lorsque 0≤M<M₂, le résultat de détermination du segment de fenêtre de détection indique la période normale ;
la classification asynchrone à trois voies consiste à effectuer la comparaison de seuils deux fois pour obtenir respectivement des quantités K₁ et K₂ des segments de fenêtre minimale anormaux détectés dans le segment de fenêtre de détection, et à comparer des relations numériques entre K₁ et chacun de M₁ et de N, ainsi que des relations numériques entre K₂ et chacun de M₂ et de N en parallèle ; et lorsque des différents résultats de détermination sont obtenus pour un même segment de fenêtre de détection, une deuxième opération logique est utilisée pour une différenciation secondaire, dans laquelle des priorités de la deuxième opération logique dans la période anormale continue avec la variation d'amplitude soudaine, la période anormale instantanée et la période normale sont dans un ordre décroissant.

6. Procédé de détection de phases temporelles pour un signal d'électroencéphalogramme selon la revendication 1, **caractérisé en ce que** :
la sélection de la phase temporelle du signal d'électroencéphalogramme en fonction du résultat de détermination d'au moins un segment de fenêtre de détection consiste à :
après avoir obtenu les résultats de détermination d'une pluralité de segments de fenêtre de détection, effectuer une détermination sur la base d'un principe du jugement k-sur-n, dans lequel dans un scénario où la spécificité de la détection est de préférence, le principe du jugement k-sur-n exige que les k segments de fenêtre de détection soient détectés de manière continue ; et
dans un scénario où la sensibilité de la détection est de préférence, le principe du jugement k-sur-n exige que les k segments de fenêtre de détection soient détectés de manière discontinue, dans lequel
n représente une quantité de segments de fenêtre de détection contenus dans le signal d'électroencéphalogramme, et k représente une quantité de segments de fenêtre de détection détectés parmi n segments de fenêtre de détection.

7. Procédé de détection de phases temporelles pour un signal d'électroencéphalogramme selon la revendication 1, **caractérisé en ce que** :
l'algorithme d'alerte précoce en fonction du coefficient de passage par zéro comprend :
diviser le signal d'électroencéphalogramme en une pluralité de fenêtres pour obtenir une pluralité de segments de données ;
extraire une valeur propre de signal pour chacun de la pluralité segments de données séparément, et entrer la valeur propre de signal dans un classificateur pour obtenir un résultat de classification de chacun de la pluralité segments de données ; et
si des résultats de classification de X segments de données consécutifs correspondent à la période anormale continue avec la variation d'amplitude progressive, déterminer que le signal d'électroencéphalogramme est dans la période anormale continue avec la variation d'amplitude progressive.

8. Procédé de détection de phases temporelles pour un signal d'électroencéphalogramme selon la revendication 7, **caractérisé en ce que** :
lorsqu'au moins deux détecteurs exécutent deux différents algorithmes de détection et que les deux différents algorithmes de détection ont des valeurs propres de signal obtenus à travers une même opération linéaire, la valeur propre de signal extraite de chacun de la pluralité de segments de données dans l'algorithme d'alerte précoce en fonction du coefficient de passage par zéro est définie comme somme des valeurs propres de signal des segments de fenêtre minimale du segment de fenêtre de détection dans l'algorithme de classification en fonction de la fenêtre minimale, les valeurs propres de signal comprenant chacune au moins l'un parmi le coefficient de passage par zéro, une longueur de ligne et une courbure.

9. Unité de détection de phases temporelles, **caractérisée en ce que** l'unité de détection de phases temporelles adopte le procédé de détection de phases temporelles pour le signal d'électroencéphalogramme selon l'une quelconque des revendications 1 à 8, et l'unité de détection de phases temporelles comprend :
un composant de détection comprenant une pluralité de détecteurs configurés pour produire des résultats de détection correspondant à différentes phases temporelles ; et
un processeur configuré pour déterminer une phase temporelle d'un signal d'électroencéphalogramme en fonction des résultats de détection correspondant aux différentes phases temporelles.

10. Système de régulation et de contrôle de boucle fermée, **caractérisé en ce que** :
l'unité de détection de phases temporelles selon la revendication 9 ; et
un module de régulation et de contrôle configuré pour effectuer une régulation et un contrôle de boucle fermée en fonction d'une phase temporelle d'un signal d'électroencéphalogramme.
